# EUROPEAN PATENT SPECIFICATION

(11) **EP 3 355 875 B1**
(45) Date of publication and mention of the grant of the patent: **29.09.2021**
(21) Application number: 16782161.0
(22) Date of filing: 30.09.2016
(51) Int. Cl.: A61K 31/122, A61K 31/351, A61K 31/4748, A61K 31/522, A61K 31/675, A61P 35/00, A61K 45/06, A61K 39/395

(54) **COMBINATION OF A BTK INHIBITOR AND A CHECKPOINT INHIBITOR FOR TREATING CANCERS**
KOMBINATION AUS EINEM BTK-INHIBITOR UND EINEM CHECKPOINT-INHIBITOR ZUR BEHANDLUNG VON KREBS
COMBINAISON D'UN INHIBITEUR DE BTK ET D'UN INHIBITEUR DE POINT DE CONTRÔLE POUR LE TRAITEMENT DE CANCERS

(30) Priority: 01.10.2015 US 201562236064 P; 17.12.2015 US 201562269007 P
(43) Date of publication of application: 08.08.2018
(73) Proprietor: Gilead Sciences, Inc., Foster City, CA 94404 (US); ONO Pharmaceutical Co., Ltd., Osaka-shi, Osaka 541-8526 (JP)
(72) Inventor: VENKATARAMAN, Sriram, Foster City, CA 94404 (US); TUMAS, Daniel, Foster City, CA 94404 (US); TANAKA, Kohei, Mishima-gun Osaka 618-8585 (JP); YASUHIRO, Tomoko, Mishima-gun Osaka 618-8585 (JP); YOSHIZAWA, Toshio, Mishima-gun Osaka 618-8585 (JP)
(74) Representative: Jones, Nicholas Andrew
(86) International application number: PCT/US2016/054731
(87) International publication number: WO 2017/059224

(56) References cited:
- WO-A1-2016/024228
- US-A1- 2015 118 222
- US-A1- 2015 125 446

## Description

### FIELD OF THE INVENTION

The present disclosure relates generally to therapeutics and compositions for treating cancers, and more specifically to the use of Bruton's Tyrosine Kinase (Btk) inhibitors in combination with one or more checkpoint inhibitors for treating cancers as specified in the claims.

### BACKGROUND

Btk inhibitors useful in treating cancers include those taught in U.S. Pat. No. 8,940,725 (Yamamoto et al.) and U.S. Pat. No. 7,514,444 (Honigberg et al.). U.S. 2015/0118222 (Levy et al.) describes the use of a Bruton's tyrosine kinase (Btk) inhibitor in combination with checkpoint inhibitors for use in treating cancers. Also, Sagiv-Barfi et al. discloses the use of the Btk inhibitor ibrutinib and an anti-PD-Ll antibody for treating cancers (PNAS 2015; E966-E972).

A promising strategy for treating cancer concerns inhibiting the protein "checkpoints" of the immune system, including the checkpoint proteins CTLA-4, PD-1, PDL1, PDL2, B7-H3, B7-H4, BTLA, HVEM, TIM-3, GAL9, LAG3, VISTA, KIR, 2B4, CD160, CGEN-15049, CHK1, CHK2, A2aR, and the B-7 family of ligands.

There remains a need for additional treatments for cancers, including solid tumor and hematological cancers.

### BRIEF SUMMARY

Provided herein is a use of a combination of a therapeutically effective amount of a Btk inhibitor and a therapeutically effective amount of a checkpoint inhibitor as specified in the claims.

The Btk inhibitor is 6-amino-9-[(3R)-1-(2-butynoyl)-3-pyrrolidinyl]-7-(4-phenoxyphenyl)-7,9-dihydro-8H-purin-8-one, or a pharmaceutically acceptable salt or hydrate thereof, and has the chemical structure:

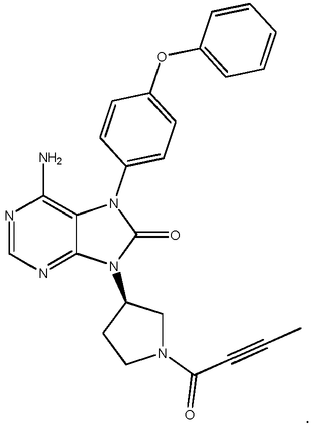

The checkpoint inhibitor is an inhibitor of PD-1. The combination is for use in treating cancer in a human in need thereof. The human subject is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

The combination is also for use in sensitizing a human who is (i) refractory to at least one chemotherapy treatment defined above, or (ii) in relapse after treatment with a chemotherapy as defined above, or both (i) and (ii).

In some variations, the Btk inhibitor is a hydrochloride salt of 6-amino-9-[(3R)-1-(2-butynoyl)-3-pyrrolidinyl]-7-(4-phenoxyphenyl)-7,9-dihydro-8H-purin-8-one, or a pharmaceutically acceptable hydrate thereof.

Provided herein are also uses of articles of manufacture and kits that comprise the Btk inhibitor and the checkpoint inhibitor described herein.

### DETAILED DESCRIPTION

The following description sets forth exemplary methods, parameters and the like. It should be recognized, however, that such description is not intended as a limitation on the scope of the present disclosure but is instead provided as a description of exemplary embodiments.

Provided herein is a combination of a therapeutically effective amount of a Btk inhibitor and a therapeutically effective amount of a checkpoint inhibitor for use in treating cancer in a human in need thereof, wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

Provided are also uses of compositions (including pharmaceutical compositions, formulations, or unit dosages), articles of manufacture and kits comprising a Btk inhibitor and a checkpoint inhibitor.

### Compounds

The Btk inhibitor is Compound A1, or a pharmaceutically acceptable salt or hydrate thereof. Compound A1 has the structure:

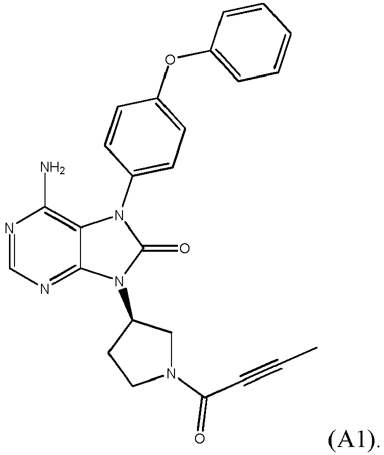

In some variations, the Btk inhibitor is a hydrochloride salt of Compound A1, or a hydrate thereof. Compound A1 may be synthesized according to the methods described in U.S. Patent No. 8,557,803 (Yamamoto et al.) and US 2014/0330015. Compound A1 may be referred to as (R)-6-amino-9-(1-(but-2-ynoyl)pyrrolidin-3-yl)-7-(4-phenoxyphenyl)-7H-purin-8(9H)-one or 6-amino-9-[(3R)-1-(2-butynoyl)-3-pyrrolidinyl]-7-(4-phenoxyphenyl)-7,9-dihydro-8H-purin-8-one.

The combination comprises a pharmaceutically effective amount of Compound A1, or a pharmaceutically acceptable salt or hydrate thereof, and a pharmaceutically effective amount of a checkpoint protein inhibitor which is an inhibitor of Programmed Death 1 (PD-1, CD279), such as nivolumab (OPDIVO®, BMS-936558, MDX1106, or MK-34775), and pembrolizumab (KEYTRUDA®, MK-3475, SCH-900475, lambrolizumab, CAS Reg. No. 1374853-91-4), as well as the PD-1 blocking agents described in U.S. Pat. No. 7,488,802, U.S. Pat. No. 7,943,743, U.S. Pat. No. 8,008,449, U.S. Pat. No. 8,168,757, U.S. Pat. No. 8,217, 149, WO 03042402, WO 2008156712, WO 2010089411, WO 2010036959, WO 2011066342, WO 2011159877, WO 2011082400, and WO 2011161699.

In another variation, the checkpoint inhibitor is an anti-PD-1 antibody. In one variation, the anti-PD-1 antibody is selected from the group of nivolumab, pembrolizumab, and lambrolizumab.

The compound names provided herein are named using ChemBioDraw Ultra 12.0. One skilled in the art understands that the compound may be named or identified using various commonly recognized nomenclature systems and symbols. By way of example, the compound may be named or identified with common names, systematic or non-systematic names. The nomenclature systems and symbols that are commonly recognized in the art of chemistry include, for example, Chemical Abstract Service (CAS), ChemBioDraw Ultra, and International Union of Pure and Applied Chemistry (IUPAC).

Also provided herein are isotopically labeled forms of compounds detailed herein. Isotopically labeled compounds have structures depicted by the formulas given herein except that one or more atoms are replaced by an atom having a selected atomic mass or mass number. Examples of isotopes that can be incorporated into compounds of the disclosure include isotopes of hydrogen, carbon, nitrogen, oxygen, phosphorous, fluorine and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl and ¹²⁵I. Various isotopically labeled compounds of the present disclosure, for example those into which radioactive isotopes such as ³H, ¹³C and ¹⁴C are incorporated, are provided. Such isotopically labeled compounds may be useful in metabolic studies, reaction kinetic studies, detection or imaging techniques, such as positron emission tomography (PET) or single-photon emission computed tomography (SPECT) including drug or substrate tissue distribution assays or in radioactive treatment of subjects (e.g. humans). Also provided for isotopically labeled compounds described herein are any pharmaceutically acceptable salts, or hydrates, as the case may be.

In some variations, the compounds disclosed herein may be varied such that from 1 to n hydrogens attached to a carbon atom is/are replaced by deuterium, in which n is the number of hydrogens in the molecule. Such compounds may exhibit increased resistance to metabolism and are thus useful for increasing the half life of the compound when administered to a mammal. See, for example, Foster, "Deuterium Isotope Effects in Studies of Drug Metabolism", Trends Pharmacol. Sci. 5(12):524-527 (1984). Such compounds are synthesized by means well known in the art, for example by employing starting materials in which one or more hydrogens have been replaced by deuterium.

Deuterium labeled or substituted therapeutic compounds of the disclosure may have improved DMPK (drug metabolism and pharmacokinetics) properties, relating to absorption, distribution, metabolism and excretion (ADME). Substitution with heavier isotopes such as deuterium may afford certain therapeutic advantages resulting from greater metabolic stability, for example increased *in vivo* half-life, reduced dosage requirements and/or an improvement in therapeutic index. An ¹⁸F labeled compound may be useful for PET or SPECT studies. Isotopically labeled compounds of this disclosure can generally be prepared by carrying out the procedures disclosed in the schemes or in the examples and preparations described below by substituting a readily available isotopically labeled reagent for a non-isotopically labeled reagent. It is understood that deuterium in this context is regarded as a substituent in the compounds provided herein.

The concentration of such a heavier isotope, specifically deuterium, may be defined by an isotopic enrichment factor. In the compounds of this disclosure any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds of this disclosure any atom specifically designated as a deuterium (D) is meant to represent deuterium.

The term "pharmaceutically acceptable" with respect to a substance refers to that substance which is generally regarded as safe and suitable for use without undue toxicity, irritation, allergic response, and the like, commensurate with a reasonable benefit/risk ratio. As used herein, by "pharmaceutically acceptable" refers to a material that is not biologically or otherwise undesirable, e.g., the material may be incorporated into a pharmaceutical composition administered to a patient without causing any significant undesirable biological effects or interacting in a deleterious manner with any of the other components of the composition in which it is contained. Pharmaceutically acceptable vehicles (e.g., carriers, adjuvants, and/or other excipients) have preferably met the required standards of toxicological and manufacturing testing and/or are included on the Inactive Ingredient Guide prepared by the U.S. Food and Drug administration.

"Pharmaceutically acceptable salt" refers to a salt of a compound that is pharmaceutically acceptable and that possesses (or can be converted to a form that possesses) the desired pharmacological activity of the parent compound. Such salts include acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic acid, camphorsulfonic acid, citric acid, ethanesulfonic acid, fumaric acid, glucoheptonic acid, gluconic acid, lactic acid, maleic acid, malonic acid, mandelic acid, methanesulfonic acid, 2-napththalenesulfonic acid, oleic acid, palmitic acid, propionic acid, stearic acid, succinic acid, tartaric acid, p-toluenesulfonic acid, trimethylacetic acid, and the like, and salts formed when an acidic proton present in the parent compound is replaced by either a metal ion, e.g., an alkali metal ion, an alkaline earth ion, or an aluminum ion; or coordinates with an organic base such as diethanolamine, triethanolamine, N-methylglucamine and the like. "Pharmaceutically acceptable salts" include, for example, salts with inorganic acids and salts with an organic acid. Examples of salts may include hydrochlorate, phosphate, diphosphate, hydrobromate, sulfate, sulfinate, nitrate, malate, maleate, fumarate, tartrate, succinate, citrate, acetate, lactate, mesylate, p-toluenesulfonate, 2-hydroxyethylsulfonate, benzoate, salicylate, stearate, and alkanoate (such as acetate, HOOC-(CH₂)ₙ-COOH where n is 0-4). In addition, if the compounds described herein are obtained as an acid addition salt, the free base can be obtained by basifying a solution of the acid salt. Conversely, if the product is a free base, an addition salt, particularly a pharmaceutically acceptable addition salt, may be produced by dissolving the free base in a suitable organic solvent and treating the solution with an acid, in accordance with conventional procedures for preparing acid addition salts from base compounds. Also included in this definition are ammonium and substituted or quaternized ammonium salts. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5. Those skilled in the art will recognize various synthetic methodologies that may be used to prepare nontoxic pharmaceutically acceptable addition salts.

The terms "effective amount", "pharmaceutically effective amount", and "therapeutically effective amount" refer to an amount that may be effective to elicit the desired biological or medical response, including the amount of a compound that, when administered to a subject for treating a disease, is sufficient to effect such treatment for the disease. The effective amount will vary depending on the compound, the disease and its severity and the age, weight, etc., of the subject to be treated. The effective amount can include a range of amounts. A pharmaceutically effective amount includes amounts of an agent which are effective when combined with other agents.

The therapeutically effective amount may vary depending on the subject, and disease or condition being treated, the weight and age of the subject, the severity of the disease or condition, and the manner of administering, which can readily be determined by one or ordinary skill in the art.

Treatment" or "treating" is an approach for obtaining beneficial or desired results including clinical results. Beneficial or desired clinical results may include one or more of the following:
(i) decreasing one more symptoms resulting from the disease;
(ii) diminishing the extent of the disease and/or stabilizing the disease (e.g., delaying the worsening of the disease);
(iii) delaying the spread of the disease;
(iv) delaying or slowing the onset or recurrence of the disease and/or the progression of the disease;
(v) ameliorating the disease state and/or providing a remission (whether partial or total) of the disease and/or decreasing the dose of one or more other medications required to treat the disease;
(vi) increasing the quality of life;
(vii) prolonging survival;
(iix) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or
(ix) relieving the disease, that is, causing the regression of clinical symptoms (e.g., ameliorating the disease state, providing partial or total remission of the disease or condition, enhancing effect of another medication, delaying the progression of the disease, increasing the quality of life, and/or prolonging survival). Without being bound to any hypothesis or theory, the methods described herein comprising one or more agents may provide unexpected treatment benefits, including but not limited to shorter treatment periods, reducing or minimizing minimal residual disease in cancers, reducing or minimizing cancer resistance, increasing survival rates, decreasing symptoms, or slowing cancer development.

"Delaying" the development of a disease or condition means to defer, hinder, slow, retard, stabilize, and/or postpone development of the disease or condition. This delay can be of varying lengths of time, depending on the history of the disease or condition, and/or subject being treated. A method that "delays" development of a disease or condition is a method that reduces probability of disease or condition development in a given time frame and/or reduces the extent of the disease or condition in a given time frame, when compared to not using the method. Such comparisons are typically based on clinical studies, using a statistically significant number of subjects. Disease or condition development can be detectable using standard methods, such as routine physical exams, mammography, imaging, or biopsy. Development may also refer to disease or condition progression that may be initially undetectable and includes occurrence, recurrence, and onset.

Reference to "about" a value or parameter herein includes (and describes) embodiments that are directed to that value or parameter per se. In certain embodiments, the term "about" includes the indicated amount ± 10%. In other embodiments, the term "about" includes the indicated amount ± 5%. In certain other embodiments, the term "about" includes the indicated amount ± 1%. Also, to the term "about X" includes description of "X". Also, the singular forms "a" and "the" include plural references unless the context clearly dictates otherwise. Thus, e.g., reference to "the compound" includes a plurality of such compounds and reference to "the assay" includes reference to one or more assays and equivalents thereof known to those skilled in the art.

### Methods of Treatment

The Btk and checkpoint inhibitors described herein may be used in a combination therapy. In one embodiment, the use for treating cancer comprises administering to a human in need thereof Compound A1 and nivolumab. In some embodiments, the use for treating cancer comprises administering to a human in need thereof Compound A1 and pembrolizumab. Without being bound to any theory or hypothesis, the combination therapy described herein may provide desired effects for cancer treatment. The results described in the present application illustrate that the combination of Compound A1 and an anti-PD-1 antibody resulted in reduced tumor volume or tumor remission. It is previously reported that B-cell lymphoma A20 would not be sensitive to the Btk inhibitor ibrutinib (Sagiv-Barfi et al. PNAS 2015; E966-E972). Sagiv-Barfi et al. and U.S. Patent Application Publication No. 2015/0118222 disclosed the use of a Btk inhibitor in combination with checkpoint inhibitors for use in treating cancers; nonetheless, both reports described the results from ibrutinib or 1-[(3R)-3-[4-Amino-3-(4-phenoxyphenyl)-1H-pyrazolo[3,4-d]pyrimidin-1-yl]piperidin-1-yl]prop-2-en-1-one as a Btk inhibitor.

### Cancer

In some embodiments, the cancer is carcinoma, sarcoma, melanoma, lymphoma or leukemia. In other embodiments, the cancer is a hematologic malignancy. In some embodiments, the cancer is leukemia (*e.g.,* chronic lymphocytic leukemia), lymphoma (*e.g.,* non-Hodgkin's lymphoma), or multiple myeloma. In some embodiments, the cancer is a B-cell cancer or B-cell malignancy. In other embodiments, the cancer is a solid tumor.

In some variations, the cancer is small lymphocytic lymphoma, non-Hodgkin's lymphoma, indolent non-Hodgkin's lymphoma (iNHL), refractory iNHL, mantle cell lymphoma, follicular lymphoma, lymphoplasmacytic lymphoma, marginal zone lymphoma, immunoblastic large cell lymphoma, lymphoblastic lymphoma, Splenic marginal zone B-cell lymphoma (+/- villous lymphocytes), nodal marginal zone lymphoma (+/- monocytoid B-cells), extranodal marginal zone B-cell lymphoma of mucosa-associated lymphoid tissue type, cutaneous T-cell lymphoma, extranodal T-cell lymphoma, anaplastic large cell lymphoma, angioimmunoblastic T-cell lymphoma, mycosis fungoides, B-cell lymphoma, diffuse large B-cell lymphoma, Mediastinal large B-cell lymphoma, Intravascular large B-cell lymphoma, Primary effusion lymphoma, small non-cleaved cell lymphoma, Burkitt's lymphoma, multiple myeloma, plasmacytoma, acute lymphocytic leukemia, T-cell acute lymphoblastic leukemia, B-cell acute lymphoblastic leukemia, B-cell prolymphocytic leukemia, acute myeloid leukemia, chronic lymphocytic leukemia, juvenile myelomonocytic leukemia, minimal residual disease, hairy cell leukemia, primary myelofibrosis, secondary myelofibrosis, chronic myeloid leukemia, myelodysplastic syndrome, myeloproliferative disease, or Waldestrom's macroglobulinemia. In some variations, the cancer is minimal residual disease (MRD). In certain variation, the MRD may be in lymphoma, leukemia, non-Hodgkin's lymphoma or indolent non-Hodgkin's lymphoma (iNHL), small lymphocytic lymphoma (SLL), chronic lymphocytic leukemia (CLL), follicular lymphoma (FL), Waldestrom's macroglobulinemia (WM), or diffuse large B-cell lymphoma (DLBCL).

In some embodiments, the B-cell malignancy is a B-cell lymphoma or a B-cell leukemia. In some variations, the B-cell malignancy is follicular lymphoma (FL), marginal zone lymphoma (MZL), small lymphocytic lymphoma (SLL), chronic lymphocytic leukemia (CLL), mantle cell lymphoma (MCL), Waldenstrom Macroglobulinemia (WM), non-germinal center B-cell lymphoma (GCB), or diffuse large B-cell lymphoma (DLBCL). In some variations, the B-cell malignancy is diffuse large B-cell lymphoma (DLBCL). In one variation, the DLBCL is activated B-cell like diffuse large B-cell lymphoma (ABC-DLBCL). In another variation, the DLBCL is germinal center B-cell like diffuse large B-cell lymphoma (GCB-DLBCL).In other variations, the B-cell malignancy is chronic lymphocytic leukemia (CLL). In other variations, the B-cell malignancy is mantle cell lymphoma (MCL). In yet other variations, the B-cell malignancy is Waldenstrom Macroglobulinemia (WM). In other variations, the cancer is pancreatic cancer, urological cancer, bladder cancer, colorectal cancer, colon cancer, breast cancer, prostate cancer, renal cancer, hepatocellular cancer, thyroid cancer, gall bladder cancer, lung cancer (e.g. non-small cell lung cancer, small-cell lung cancer), ovarian cancer, cervical cancer, gastric cancer, endometrial cancer, esophageal cancer, head and neck cancer, melanoma, neuroendocrine cancer, CNS cancer, brain tumors (*e.g.,* glioma, anaplastic oligodendroglioma, adult glioblastoma multiforme, and adult anaplastic astrocytoma), bone cancer, soft tissue sarcoma, retinoblastomas, neuroblastomas, peritoneal effusions, malignant pleural effusions, mesotheliomas, Wilms tumors, trophoblastic neoplasms, hemangiopericytomas, Kaposi's sarcomas, myxoid carcinoma, round cell carcinoma, squamous cell carcinomas, esophageal squamous cell carcinomas, oral carcinomas, cancers of the adrenal cortex, or ACTH-producing tumors.

### Subject

The human in need thereof may be an individual who has or is suspected of having a cancer. In some of variations, the human is at risk of developing a cancer (*e.g.,* a human who is genetically or otherwise predisposed to developing a cancer) and who has or has not been diagnosed with the cancer. As used herein, an "at risk" subject is a subject who is at risk of developing cancer (e.g., a hematologic malignancy). The subject may or may not have detectable disease, and may or may not have displayed detectable disease prior to the treatment methods described herein. An at risk subject may have one or more so-called risk factors, which are measurable parameters that correlate with development of cancer, such as described herein. A subject having one or more of these risk factors has a higher probability of developing cancer than an individual without these risk factor(s).

These risk factors may include, for example, age, sex, race, diet, history of previous disease, presence of precursor disease, genetic (*e.g.,* hereditary) considerations, and environmental exposure. In some embodiments, a human at risk for cancer includes, for example, a human whose relatives have experienced this disease, and those whose risk is determined by analysis of genetic or biochemical markers. Prior history of having cancer may also be a risk factor for instances of cancer recurrence.

In some embodiments, the combination of a Btk inhibitor and a checkpoint inhibitor as described herein is for use in treating a human who exhibits one or more symptoms associated with cancer (*e.g.,* a hematologic malignancy). In some embodiments, the human is at an early stage of cancer. In other embodiments, the human is at an advanced stage of cancer.

In some embodiments, the combination of a Btk inhibitor and a checkpoint inhibitor as described herein is for use in treating a human who is undergoing one or more standard therapies for treating cancer (*e.g.,* a hematologic malignancy), such as chemotherapy, radiotherapy, immunotherapy, and/or surgery. Thus, in some foregoing embodiments, the combination of a Btk inhibitor and a checkpoint inhibitor, as described herein, may be administered before, during, or after administration of chemotherapy, radiotherapy, immunotherapy, and/or surgery.

The combination of a Btk inhibitor and a checkpoint inhibitor as described herein is for use in treating a human who is "refractory" to a cancer treatment or who is in "relapse" after treatment for cancer (*e.g.,* a hematologic malignancy). A subject "refractory" to an anti-cancer therapy means they do not respond to the particular treatment, also referred to as resistant. The cancer may be resistant to treatment from the beginning of treatment, or may become resistant during the course of treatment, for example after the treatment has shown some effect on the cancer, but not enough to be considered a remission or partial remission. A subject in "relapse" means that the cancer has returned or the signs and symptoms of cancer have returned after a period of improvement, e.g. after a treatment has shown effective reduction in the cancer, such as after a subject is in remission or partial remission.

The human is (i) refractory to at least one anti-cancer therapy, or (ii) in relapse after treatment with at least one anti-cancer therapy, or both (i) and (ii). In some embodiments, the human is refractory to at least two, at least three, or at least four anti-cancer therapies (including, for example, standard or experimental chemotherapies).

In another aspect, provided is a combination of a Btk inhibitor and a checkpoint inhibitor as described herein for use in sensitizing a human who is (i) refractory to at least one chemotherapy treatment, or (ii) in relapse after treatment with chemotherapy, or both (i) and (ii). A human who is sensitized is a human who is responsive to the treatment involving administration of a Btk inhibitor in combination with a checkpoint inhibitor, as described herein, or who has not developed resistance to such treatment.

In an embodiment, provided herein is a combination of a Btk inhibitor and a checkpoint inhibitor as described herein for use in treating a human for a cancer, with comorbidity, wherein the treatment is also effective in treating the comorbidity. A "comorbidity" to cancer is a disease that occurs at the same time as the cancer.

For chronic lymphocytic leukemia the prior treatments a human may have received include regimens of:
a) fludarabine (Fludara ®);
b) rituximab (Rituxan®);
c) rituximab (Rituxan ®) combined with fludarabine (sometimes abbreviated as FR);
d) cyclophosphamide (Cytoxan®) combined with fludarabine; cyclophosphamide combined with rituximab and fludarabine (sometimes abbreviated as FCR);
e) cyclophosphamide combined with vincristine and prednisone (sometimes abbreviated as CVP);
f) cyclophosphamide combined with vincristine, prednisone, and rituximab;
g) combination of cyclophosphamide, doxorubicin, vincristine (Oncovin), and prednisone (sometimes referred to as CHOP);
h) Chlorambucil combined with prednisone, rituximab, obinutuzumab, or ofatumumab
i) pentostatin combined with cyclophosphamide and rituximab (sometimes abbreviated as PCR);
j) bendamustine (Treanda®) combined with rituximab (sometimes abbreviated as BR);
k) alemtuzumab (Campath®);
l) fludarabine plus cyclophosphamide, bendamustine, or chlorambucil; and
m) fludarabine plus cyclophosphamide, bendamustine, or chlorambucil, combined with an anti-CD20 antibody, such as rituximab, ofatumumab, or obinutuzumab.

### Therapeutically Effective Amounts

In some variations, a therapeutically effective amount refers to an amount that is sufficient to effect treatment, as defined below, when administered to a subject (*e.g.,* a human) in need of such treatment. The therapeutically effective amount will vary depending upon the subject and disease condition being treated, the weight and age of the subject, the severity of the disease condition, the manner of administration and the like, which can readily be determined by one of ordinary skill in the art. For example, in one variation, a therapeutically effective amount of Compound A1, or a pharmaceutically acceptable salt or hydrate thereof, is an amount sufficient to modulate Btk expression, and thereby treat a human suffering an indication, or to ameliorate or alleviate the existing symptoms of the indication. In one variation, a therapeutically effective amount of a checkpoint inhibitor is an amount sufficient to modulate activity of one or more checkpoint proteins, and thereby treat a human suffering an indication, or to ameliorate or alleviate the existing symptoms of the indication.

In another variation, the therapeutically effective amount of the Btk inhibitor Compound A1, or a pharmaceutically acceptable salt or hydrate thereof, may be an amount sufficient to decrease a symptom of a disease or condition responsive to inhibition of Btk activity.

In one variation, the therapeutically effective amount of the Btk inhibitor is a dose corresponding to 1 nmol to 10,000 nmol of the Btk inhibitor used in an apoptosis assay run with 10% serum which approximately relates to a blood plasma concentration of 500 nmol to 2500 nmol of the Btk inhibitor. In one variation, the therapeutically effective amount of the checkpoint inhibitor is a dose corresponding to 1 nmol to 200 nmol of the checkpoint inhibitor used in an apoptosis assay run with 10% serum. Specific examples include 3 nM, 5 nM, 10 nM, 20 nM and 30 nM concentrations when combined with a checkpoint inhibitor.

The therapeutically effective amount of the Btk and checkpoint inhibitors may also be determined based on data obtained from assays known in the art, including for example, the apoptosis assays or anti-tumor efficacy studies. In one variation, the therapeutically effective amount of the Btk inhibitor in a human is a dose of from 1 mg to 200 mg. In another embodiment the Btk in a human is administered at a dose of from 10 mg to 200 mg. In another embodiment the Btk in a human is administered at a dose of from 20 mg to 160 mg. In other separate embodiments the Btk inhibitor is administered to a human at a dose of: a) from 10 mg to 100 mg, b) from 50 mg to 175 mg, c) from 20 mg to 150 mg, d) from 75 mg to 100 mg, and e) from 100 mg to 200 mg. Individual doses of the Btk inhibitor that may be administered to a human in need thereof include individual doses of 1mg, 5 mg, 10 mg, 20 mg, 30 mg, 40 mg, 50 mg, 60 mg, 70 mg, 75 mg, 80 mg, 901 mg, 100 mg, 110 mg, 120 mg, 130 mg, 140 mg, 150 mg, 160 mg, 170 mg, 175 mg, and 200 mg. The doses of the Btk inhibitor may be administered as determined by a medical professional and may be administered once daily or may be delivered twice daily, three times daily, or four times daily.

In another variation, the Btk inhibitor Compound A1, or a pharmaceutically acceptable salt or hydrate thereof, is administered to the human at a dose resulting in 50%, 55%, 60%, 65%, 70%, 75%, 80%, 90%, 95%, or 99% Btk target inhibition. In another variation, the checkpoint inhibitor is administered to the human at a dose resulting in 50%, 55%, 60%, 65%, 70%, 75%, 80%, 90%, 95%, or 99% checkpoint protein target inhibition.

In some variations, the Btk inhibitor Compound A1, or a pharmaceutically acceptable salt or hydrate thereof, is administered to the human at a dose between 40 mg and 1200 mg, between 40 mg and 800 mg, between 40 mg and 600 mg, between 40 mg and 40 mg, 100 mg, 100 mg, 200 mg, 300 mg, 400 mg, 500 mg, 600 mg, 700 mg, or 800 mg. In some variations, the Checkpoint inhibitor is administered to the human at a dose between 20 to 600 mg, between 20 to 400 mg, between 20 to 200 mg, 20 mg, 50 mg, 100 mg, 200 mg, 300 mg, 400 mg, 500 mg, 600 mg, 700 mg, or 800 mg.

The therapeutically effective amount of the Btk and checkpoint inhibitors may be provided in a single dose or multiple doses to achieve the desired treatment endpoint. As used herein, "dose" refers to the total amount of an active ingredient to be taken each time by a human. The dose administered, for example for oral administration described above, may be administered once weekly, once daily (QD), twice daily (BID), three times daily, four times daily, or more than four times daily. In some embodiments, the Btk and/or the checkpoint inhibitors may be administered once daily. In some embodiments, the Btk and/or the checkpoint inhibitors may be administered twice daily. In some embodiments, the checkpoint inhibitors may be administered once weekly or with a frequency that can vary between daily, every other day, once every 5 days, daily for 1, 2, 3, 4, 5, 6 or 7 days and then weekly or with a regimen that can combine these different frequencies and doses to result in a final dose and regimen that is tolerated and efficacious. The regimens herein are intended to encompass those in which the Btk inhibitor and checkpoint protein inhibitor are administered to the human in need thereof at the same or different times.

In one variation, the therapeutically effective amount of the checkpoint inhibitor is a dose that provides sufficient receptor occupancy to elicit antigen-specific T cell responses. Individual doses of the checkpoint inhibitor (anti-PD-1 antibodies) may be administered to a human in need thereof. The doses of the checkpoint inhibitor may be administered as determined by a medical professional and may be administered once every two, three, or four weeks and may be continued to be delivered in these period cycles continuously for up to and more than 3 years._ In another embodiment, the amount of antibody administered varies with each dose. For example, the maintenance (or follow-on) dose of the antibody can be higher or the same as the loading dose which is first administered. In another embodiment, the maintenance dose of the antibody can be lower or the same as the loading dose. In other embodiments, the checkpoint inhibitor antibody is administered once per week, once every or three two weeks, once per month or as long as a clinical benefit is observed or until there is a complete response, confirmed progressive disease or unmanageable toxicity.

In another exemplary embodiment, a checkpoint inhibitor antibody may preferably be administered at 0.3-10 mg/kg, or the maximum tolerated dose, administered every two, three, four weeks or every six weeks. In another embodiment, the dose of the checkpoint inhibitor antibody is a flat-fixed dose. In some embodiments, 1 mg/kg is administered every two weeks. In other embodiments, 2 mg/kg is administered every three weeks. In yet other embodiments, 3mg/kg is administered every two weeks. In some embodiments, the dose is administered as an intravenous infusion over a 60 minute period. In other embodiments, the dose is administered as an intravenous infusion over a 30 minute period. In another embodiment, the dose of the checkpoint inhibitor antibody is varied over time. For example, the anti-PD-1 antibody may be initially administered at a high dose and may be lowered over time. In another embodiment, the checkpoint inhibitor antibody is initially administered at a low dose and increased over time. Alternatively, through an escalating dosage regimen, the checkpoint inhibitor antibody may be administered including administering a first dosage at 1 mg/kg or 3 mg/kg, a second dosage at 5 mg/kg, and a third dosage at 10 mg/kg. Alternatively, the escalating dosage regimen includes administering a first dosage of checkpoint inhibitor antibody at 5 mg/kg and a second dosage at 10 mg/kg. Another stepwise escalating dosage regimen may include administering a first dosage of checkpoint inhibitor antibody 1 mg/kg, a second dosage of 3 mg/kg, a third dosage of 3 mg/kg, a fourth dosage of 5 mg/kg, and a fifth dosage of 10 mg/kg. In another aspect, a stepwise escalating dosage regimen may include administering a first dosage of 3 mg/kg, a second dosage of 5 mg/kg, and a third dosage of 10 mg/kg (Postow et al., N Engl J Med. 2015; 372(21):2006-17) In another embodiment, a cycle of administration is eight weeks, which can be repeated, as necessary. In another embodiment, the treatment consists of up to 12 cycles.

### Administration

The Btk inhibitor Compound A1, and the checkpoint inhibitors described herein may be administered using any suitable methods known in the art. For example, the compounds may be administered bucally, ophthalmically, orally, osmotically, parenterally (intramuscularly, intraperitoneally intrasternally, intravenously, subcutaneously), rectally, topically, transdermally, or vaginally. In certain embodiment, the Btk inhibitor is administered orally. In certain other embodiment, the checkpoint inhibitor is administered intravenously. In one embodiment, the Btk inhibitor Compound A1 or hydrochloride salt thereof, is administered orally, once a day, to a subject in need thereof at a dose of 20 mg, 40 mg, 80 mg, or 150mg. In some embodiments, the Btk inhibitor Compound A1 or hydrochloride salt thereof, is administered orally, twice a day, to a subject at a dose of 20 mg, 40mg, or 75 mg. In other embodiments, the checkpoint inhibitor is nivolumab, which is administered via intravenous injection, once every two weeks, to a subject in need thereof at a dose of 1 mg/kg, 2 mg/kg, or 3 mg/kg. In additional embodiments, the checkpoint inhibitor is pembrolizumab, which is administered via intravenous injection, once every three weeks, to a subject in need thereof at a dose of 2 mg/kg, 100 mg, or 200 mg. Further, in certain variations, the Btk inhibitor described herein may be administered prior, after or concurrently with the checkpoint inhibitors described herein.

### Pharmaceutical Compositions

The Btk and checkpoint inhibitors may be administered in the form of pharmaceutical compositions. For example, in some variations, the Btk inhibitor described herein may be present in a pharmaceutical composition comprising the Btk inhibitor, and at least one pharmaceutically acceptable vehicle. In some variations, the checkpoint inhibitors described herein may be present in a pharmaceutical composition comprising the checkpoint inhibitor, and at least one pharmaceutically acceptable vehicle. Pharmaceutically acceptable vehicles may include pharmaceutically acceptable carriers, adjuvants and/or excipients, and other ingredients can be deemed pharmaceutically acceptable insofar as they are compatible with other ingredients of the formulation and not deleterious to the recipient thereof.

This disclosure therefore provides pharmaceutical compositions that contain the Btk and checkpoint inhibitors as described herein, and one or more pharmaceutically acceptable vehicle, such as excipients, carriers, including inert solid diluents and fillers, diluents, including sterile aqueous solution and various organic solvents, permeation enhancers, solubilizers and adjuvants. The pharmaceutical compositions may be administered alone or in combination with other therapeutic agents. Such compositions are prepared in a manner well known in the pharmaceutical art (see, e.g., Remington's Pharmaceutical Sciences, Mace Publishing Co., Philadelphia, PA 17th Ed. (1985); and Modern Pharmaceutics, Marcel Dekker, Inc. 3rd Ed. (G.S. Banker & C.T. Rhodes, Eds.).

The pharmaceutical compositions may be administered in either single or multiple doses by any of the accepted modes of administration of agents having similar utilities, including rectal, buccal, intranasal and transdermal routes, by intra-arterial injection, intravenously, intraperitoneally, parenterally, intramuscularly, subcutaneously, orally, topically, as an inhalant, or via an impregnated or coated device such as a stent, for example, or an artery-inserted cylindrical polymer.

In some embodiments, the pharmaceutical compositions described herein are formulated in a unit dosage form. The term "unit dosage forms" refers to physically discrete units suitable as unitary dosages for human subjects, each unit containing a predetermined quantity of active material calculated to produce the desired therapeutic effect, in association with a suitable pharmaceutical excipient. In some variations, the pharmaceutical compositions described herein are in the form of a tablet, capsule, or ampoule.

In certain embodiments, the Btk inhibitor described herein, Compound A1, or a pharmaceutically acceptable salt or hydrate thereof, is formulated as a tablet. In some variations, such tablet may comprise a hydrochloride salt of Compound A1. Such tablet comprising Compound A1, for example, may be prepared by suitable methods known in the art, such as spray-drying and granulation (*e.g.,* dry granulation).

### Additional Therapeutic Agents

In the present disclosure, in some aspects, the combination described herein may be further used or combined with a chemotherapeutic agent, an immunotherapeutic agent, a radiotherapeutic agent, an anti-neoplastic agent, an anti-cancer agent, an anti-proliferation agent, an anti-fibrotic agent, an anti-angiogenic agent, a therapeutic antibody, or any combination thereof.

Chemotherapeutic agents may be categorized by their mechanism of action into, for example, the following groups: anti-metabolites/anti-cancer agents, such as pyrimidine analogs (floxuridine, capecitabine, and cytarabine); purine analogs, folate antagonists (such as pralatrexate) and related inhibitors antiproliferative/antimitotic agents including natural products such as vinca alkaloid (vinblastine, vincristine) and microtubule such as taxane (paclitaxel, docetaxel), vinblastin, nocodazole, epothilones, vinorelbine and navelbine, epidipodophyllotoxins (etoposide, teniposide); DNA damaging agents (actinomycin, amsacrine, busulfan, carboplatin, chlorambucil, cisplatin, cyclophosphamide, Cytoxan, dactinomycin, daunorubicin, doxorubicin, epirubicin, iphosphamide, melphalan, merchlorehtamine, mitomycin, mitoxantrone, nitrosourea, procarbazine, taxol, taxotere, teniposide, etoposide, triethylenethiophosphoramide); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; asparaginase stimulators, such as crisantaspase (Erwinase®) and GRASPA (ERY-001, ERY-ASP); antiproliferative/antimitotic alkylating agents such as nitrogen mustards cyclophosphamide and analogs, melphalan, chlorambucil), and (hexamethylmelamine and thiotepa), alkyl nitrosoureas (BCNU) and analogs, streptozocin), trazenes-dacarbazinine (DTIC); antiproliferative/antimitotic antimetabolites such as folic acid analogs (methotrexate); platinum coordination complexes (cisplatin, oxiloplatinim, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel; antimigratory agents; antisecretory agents (breveldin); immunosuppressives tacrolimus sirolimus azathioprine, mycophenolate; compounds (TNP-470, genistein) and growth factor inhibitors (vascular endothelial growth factor inhibitors, fibroblast growth factor inhibitors); angiotensin receptor blocker, nitric oxide donors; anti-sense oligonucleotides; antibodies (trastuzumab, rituximab); cell cycle inhibitors and differentiation inducers (tretinoin); inhibitors, topoisomerase inhibitors (doxorubicin (adriamycin), daunorubicin, dactinomycin, eniposide, epirubicin, etoposide, idarubicin, irinotecan and mitoxantrone, topotecan, irinotecan), corticosteroids (cortisone, dexamethasone, hydrocortisone, methylpednisolone, prednisone, and prednisolone); growth factor signal transduction kinase inhibitors; dysfunction inducers, toxins such as Cholera toxin, ricin, Pseudomonas exotoxin, Bordetella pertussis adenylate cyclase toxin, or diphtheria toxin, and caspase activators; and chromatin.

As used herein, other agents may include smoothened (SMO) receptor inhibitors, such as Odomzo (sonidegib, formerly LDE-225), LEQ506, vismodegib (GDC-0449), BMS-833923, glasdegib (PF-04449913), LY2940680, and itraconazole; interferon alpha ligand modulators, such as interferon alfa-2b, interferon alpha-2a biosimilar (Biogenomics), ropeginterferon alfa-2b (AOP-2014, P-1101, PEGIFN alpha-2b), Multiferon (Alfanative, Viragen), interferon alpha 1b, Roferon-A (Canferon, Ro-25-3036), interferon alfa-2a follow-on biologic (Biosidus)(Inmutag, Inter 2A), interferon alfa-2b follow-on biologic (Biosidus - Bioferon, Citopheron, Ganapar)(Beijing Kawin Technology - Kaferon)(AXXO - interferon alfa-2b), Alfaferone, pegylated interferon alpha-lb, peginterferon alfa-2b follow-on biologic (Amega), recombinant human interferon alpha-lb, recombinant human interferon alpha-2a, recombinant human interferon alpha-2b, veltuzumab-IFN alpha 2b conjugate, Dynavax (SD-101), and interferon alfa-nl (Humoferon, SM-10500, Sumiferon); interferon gamma ligand modulators, such as interferon gamma (OH-6000, Ogamma 100); Complement C3 modulators, such as Imprime PGG; IL-6 receptor modulators, such as tocilizumab, siltuximab, AS-101 (CB-06-02, IVX-Q-101); Telomerase modulators, such as tertomotide (GV-1001, HR-2802, Riavax) and imetelstat (GRN-163, JNJ-63935937); DNA methyltransferases inhibitors, such as temozolomide (CCRG-81045), decitabine, guadecitabine (S-110, SGI-110), KRX-0402, and azacitidine; DNA gyrase inhibitors, such as pixantrone and sobuzoxane.

As used herein the term "chemotherapeutic agent" or "chemotherapeutic" (or "chemotherapy," in the case of treatment with a chemotherapeutic agent) is meant to encompass any non-proteinaceous (i.e, non-peptidic) chemical compound useful in the treatment of cancer. Examples of chemotherapeutic agents include alkylating agents such as thiotepa and cyclophosphamide (CYTOXAN®); alkyl sulfonates such as busulfan, improsulfan and piposulfan; aziridines such as benzodopa, carboquone, meturedopa, and uredopa; ethylenimines and methylamelamines including alfretamine, triemylenemelamine, triethylenephosphoramide, triethylenethiophosphoramide and trimemylolomelamine; acetogenins (especially bullatacin and bullatacinone); a camptothecin (including synthetic analogue topotecan); bryostatin; callystatin; CC-1065 (including its adozelesin, carzelesin and bizelesin synthetic analogues); cryptophycins (articularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, foremustine, lomustine, nimustine, ranimustine; antibiotics such as the enediyne antibiotics (e.g., calicheamicin, especially calicheamicin gammaII and calicheamicin phiI1, see, e.g., Agnew, Chem. Intl. Ed. Engl, 33:183-186 (1994); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carrninomycin, carzinophilin, chromomycins, dactinomycin, daunorubicin, detorubicin, 6-diazo-5-oxo-L-norleucine, doxorubicin (Adramycin.TM.) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as demopterin, methotrexate, pteropterin, trimetrexate; purine analogs such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replinisher such as frolinic acid; aceglatone; aldophosphamide glycoside; aminolevulinic acid; eniluracil; amsacrine; hestrabucil; bisantrene; edatraxate; defofamine; demecolcine; diaziquone; elformthine; elliptinium acetate; an epothilone; etoglucid; gallium nitrate; hydroxyurea; lentinan; leucovorin; lonidamine; maytansinoids such as maytansine and ansamitocins; mitoguazone; mitoxantrone; mopidamol; nitracrine; pentostatin; phenamet; pirarubicin; losoxantrone; fluoropyrimidine; folinic acid; podophyllinic acid; 2-ethylhydrazide; procarbazine; PSK; razoxane; rhizoxin; sizofiran; spirogermanium; tenuazonic acid; triaziquone; 2,2',2"-tricUorotriemylamine; trichothecenes (especially T-2 toxin, verracurin A, roridin A and anguidine); urethane; vindesine; dacarbazine; mannomustine; mitobronitol; mitolactol; pipobroman; gacytosine; arabinoside ("Ara-C"); cyclophosphamide; thiopeta; taxoids, e.g., paclitaxel (TAXOL®, Bristol Meyers Squibb Oncology, Princeton, N.J.) and docetaxel (TAXOTERE®, Rhone-Poulenc Rorer, Antony, France); chlorambucil; gemcitabine (GEMZAR®); 6-thioguanine; mercaptopurine; methotrexate; platinum analogs such as cisplatin and carboplatin; vinblastine; platinum; etoposide (VP-16); ifosfamide; mitroxantrone; vancristine; vinorelbine (NAVELBINE®); novantrone; teniposide; edatrexate; daunomycin; aminopterin; xeoloda; ibandronate; CPT-11; topoisomerase inhibitor RFS 2000; difluoromethylornithine (DMFO); retinoids such as retinoic acid; capecitabine; FOLFIRI (fluorouracil, leucovorin, and irinotecan) and pharmaceutically acceptable salts, acids or derivatives of any of the above.

Also included in the definition of "chemotherapeutic agent" are anti-hormonal agents that act to regulate or inhibit hormone action on tumors such as anti-estrogens and selective estrogen receptor modulators (SERMs), including, for example, tamoxifen (including NOLVADEX™), raloxifene, droloxifene, 4-hydroxytamoxifen, trioxifene, keoxifene, LY117018, onapristone, and toremifene (FARESTON®); inhibitors of the enzyme aromatase, which regulates estrogen production in the adrenal glands, such as, for example, 4(5)-imidazoles, aminoglutethimide, megestrol acetate (MEGACE®), exemestane, formestane, fadrozole, vorozole (RIVISOR®), letrozole (FEMARA®), and anastrozole (ARIMIDEX®); and anti-androgens such as flutamide, nilutamide, bicalutamide, leuprohde, and goserelin; and pharmaceutically acceptable salts, acids or derivatives of any of the above.

The anti-angiogenic agents include, but are not limited to, retinoid acid and derivatives thereof, 2-methoxyestradiol, ANGIOSTATIN®, ENDOSTATIN®, suramin, squalamine, tissue inhibitor of metalloproteinase-1, tissue inhibitor of metalloproternase-2, plasminogen activator inhibitor-1, plasminogen activator inbibitor-2, cartilage-derived inhibitor, paclitaxel (nab-paclitaxel), platelet factor 4, protamine sulphate (clupeine), sulphated chitin derivatives (prepared from queen crab shells), sulphated polysaccharide peptidoglycan complex (sp-pg), staurosporine, modulators of matrix metabolism, including for example, proline analogs ((1-azetidine-2-carboxylic acid (LACA), cishydroxyproline, d,I-3,4-dehydroproline, thiaproline, .alpha.-dipyridyl, beta-aminopropionitrile fumarate, 4-propyl-5-(4-pyridinyl)-2(3h)-oxazolone; methotrexate, mitoxantrone, heparin, interferons, 2 macroglobulin-serum, chimp-3, chymostatin, beta-cyclodextrin tetradecasulfate, eponemycin; fumagillin, gold sodium thiomalate, d-penicillamine (CDPT), beta-1-anticollagenase-serum, alpba-2-antiplasmin, bisantrene, lobenzarit disodium, n-2-carboxyphenyl-4-chloroanthronilic acid disodium or "CCA", thalidomide; angiostatic steroid, cargboxynaminolmidazole; metalloproteinase inhibitors such as BB94. Other anti-angiogenesis agents include antibodies, preferably monoclonal antibodies against these angiogenic growth factors: beta-FGF, alpha-FGF, FGF-5, VEGF isoforms, VEGF-C, HGF/SF and Ang-1/Ang-2. See Ferrara N. and Alitalo, K. "Clinical application of angiogenic growth factors and their inhibitors" (1999) Nature Medicine 5:1359-1364.

The anti-fibrotic agents include, but are not limited to, the compounds such as beta-aminoproprionitrile (BAPN), as well as the compounds disclosed in U.S. Pat. No. 4,965,288 to Palfreyman, et al., issued Oct. 23, 1990, entitled "Inhibitors of lysyl oxidase," relating to inhibitors of lysyl oxidase and their use in the treatment of diseases and conditions associated with the abnormal deposition of collagen; U.S. Pat. No. 4,997,854 to Kagan, et al., issued Mar. 5, 1991, entitled "Anti-fibrotic agents and methods for inhibiting the activity of lysyl oxidase in situ using adjacently positioned diamine analogue substrate," relating to compounds which inhibit LOX for the treatment of various pathological fibrotic states. Further exemplary inhibitors are described in U.S. Pat. No. 4,943,593 to Palfreyman, et al., issued Jul. 24, 1990, entitled "Inhibitors of lysyl oxidase," relating to compounds such as 2-isobutyl-3-fluoro-, chloro-, or bromo-allylamine; as well as, e.g., U.S. Pat. No. 5,021,456; U.S. Pat. No. 5,5059,714; U.S. Pat. No. 5,120,764; U.S. Pat. No. 5,182,297; U.S. Pat. No. 5,252,608 (relating to 2-(1-naphthyloxymemyl)-3-fluoroallylamine); and U.S. Patent Application No. 2004/0248871. Exemplary anti-fibrotic agents also include the primary amines reacting with the carbonyl group of the active site of the lysyl oxidases, and more particularly those which produce, after binding with the carbonyl, a product stabilized by resonance, such as the following primary amines: emylenemamine, hydrazine, phenylhydrazine and their derivatives, semicarbazide, and urea derivatives, aminonitriles, such as beta-aminopropionitrile (BAPN), or 2-nitroethylamine, unsaturated or saturated haloamines, such as 2-bromo-ethylamine, 2-chloroethylamine, 2-trifluoroethylamine, 3-bromopropylamine, p-halobenzylamines, selenohomocysteine lactone. Also, the anti-fibrotic agents are copper chelating agents, penetrating or not penetrating the cells. Exemplary compounds include indirect inhibitors such compounds blocking the aldehyde derivatives originating from the oxidative deamination of the lysyl and hydroxylysyl residues by the lysyl oxidases, such as the thiolamines, in particular D-penicillamine, or its analogues such as 2-amino-5-mercapto-5-methylhexanoic acid, D-2-amino-3-methyl-3-((2-acetamidoethyl)dithio)butanoic acid, p-2-amino-3-methyl-3-((2-aminoethyl)dithio)butanoic acid, sodium-4-((p-1-dimethyl-2-amino-2-carboxyethyl)dithio)butane sulphurate, 2-acetamidoethyl-2-acetamidoethanethiol sulphanate, sodium-4-mercaptobutanesulphinate trihydrate.

The immunotherapeutic agents include and are not limited to therapeutic antibodies suitable for treating patients; such as abagovomab, adecatumumab, afutuzumab, alemtuzumab, altumomab, amatuximab, anatumomab, arcitumomab, bavituximab, bectumomab, bevacizumab, bivatuzumab, blinatumomab, brentuximab, cantuzumab, catumaxomab, cetuximab, citatuzumab, cixutumumab, clivatuzumab, conatumumab, daratumumab, drozitumab, duligotumab, dusigitumab, detumomab, dacetuzumab, dalotuzumab, ecromeximab, elotuzumab, ensituximab, ertumaxomab, etaracizumab, farietuzumab, ficlatuzumab, figitumumab, flanvotumab, futuximab, ganitumab, gemtuzumab, girentuximab, glembatumumab, ibritumomab, igovomab, imgatuzumab, indatuximab, inotuzumab, intetumumab, ipilimumab, iratumumab, labetuzumab, lexatumumab, lintuzumab, lorvotuzumab, lucatumumab, mapatumumab, matuzumab, milatuzumab, minretumomab, mitumomab, moxetumomab, narnatumab, naptumomab, necitumumab, nimotuzumab, nofetumomabn, ocaratuzumab, ofatumumab, olaratumab, onartuzumab, oportuzumab, oregovomab, panitumumab, parsatuzumab, patritumab, pemtumomab, pertuzumab, pintumomab, pritumumab, racotumomab, radretumab, rilotumumab, rituximab, robatumumab, satumomab, sibrotuzumab, siltuximab, simtuzumab, solitomab, tacatuzumab, taplitumomab, tenatumomab, teprotumumab, tigatuzumab,tocilizumab, tositumomab, trastuzumab, tucotuzumab, ublituximab, veltuzumab, vorsetuzumab, votumumab, zalutumumab, CC49 and 3F8. The exemplified therapeutic antibodies may be further labeled or combined with a radioisotope particle, such as indium In 111, yttrium Y 90, iodine 1-131.

In a certain embodiments, the additional therapeutic agent is a nitrogen mustard alkylating agent. Nonlimiting examples of nitrogen mustard alkylating agents include chlorambucil.

Some chemotherapy agents suitable for treating lymphoma or leukemia include aldesleukin, alvocidib, antineoplaston AS2-1, antineoplaston A10, anti-thymocyte globulin, amifostine trihydrate, aminocamptothecin, arsenic trioxide, beta alethine, BCL-2 family protein inhibitor ABT-263, ABT-199, ABT-737, BMS-345541, bortezomib (VELCADE®), bryostatin 1, busulfan, carboplatin, campath-1H, CC-5103, carmustine, caspofungin acetate, clofarabine, cisplatin, Cladribine (Leustarin), Chlorambucil (Leukeran), Curcumin, cyclosporine, Cyclophosphamide (Cyloxan, Endoxan, Endoxana, Cyclostin), cytarabine, denileukin diftitox, dexamethasone, DT PACE, docetaxel, dolastatin 10, Doxorubicin (ADRIAMYCIN®, Adriblastine), doxorubicin hydrochloride, enzastaurin, epoetin alfa, etoposide, Everolimus (RAD001), fenretinide, filgrastim, melphalan, mesna, Flavopiridol, Fludarabine (Fludara), Geldanamycin (17-AAG), ifosfamide, irinotecan hydrochloride, ixabepilone, Lenalidomide (REVLIMID®, CC-5013), lymphokine-activated killer cells, melphalan, methotrexate, mitoxantrone hydrochloride, motexafin gadolinium, mycophenolate mofetil, nelarabine, oblimersen (Genasense) Obatoclax (GX15-070), oblimersen, octreotide acetate, omega-3 fatty acids, oxaliplatin, paclitaxel, PD0332991, PEGylated liposomal doxorubicin hydrochloride, pegfilgrastim, Pentstatin (Nipent), perifosine, Prednisolone, Prednisone, R-roscovitine (Selicilib, CYC202), recombinant interferon alfa, recombinant interleukin-12, recombinant interleukin-11, recombinant flt3 ligand, recombinant human thrombopoietin, rituximab, sargramostim, sildenafil citrate, simvastatin, sirolimus, Styryl sulphones, tacrolimus, tanespimycin, Temsirolimus (CC1-779), Thalidomide, therapeutic allogeneic lymphocytes, thiotepa, tipifarnib, VELCADE® (bortezomib or PS-341), Vincristine (Oncovin), vincristine sulfate, vinorelbine ditartrate, Vorinostat (SAHA), vorinostat, and FR (fludarabine, rituximab), CHOP (cyclophosphamide, doxorubicin, vincristine, prednisone), CVP (cyclophosphamide, vincristine and prednisone), FCM (fludarabine, cyclophosphamide, mitoxantrone), FCR (fludarabine, cyclophosphamide, rituximab), hyperCVAD (hyperfractionated cyclophosphamide, vincristine, doxorubicin, dexamethasone, methotrexate, cytarabine), ICE (iphosphamide, carboplatin and etoposide), MCP (mitoxantrone, chlorambucil, and prednisolone), R-CHOP (rituximab plus CHOP), R-CVP (rituximab plus CVP), R-FCM (rituximab plus FCM), R-ICE (rituximab-ICE), and R-MCP (R-MCP).

In one embodiment, the combination described herein may be used or combined with one or more additional therapeutic agents. The one or more therapeutic agents include, but are not limited to, an inhibitor of Abl, activated CDC kinase (ACK), adenosine A2B receptor (A2B), apoptosis signal-regulating kinase (ASK), Auroa kinase, BET-bromodomain (BRD) such as BRD4, c-Kit, c-Met, CDK-activating kinase (CAK), calmodulin-dependent protein kinase (CaMK), cyclin-dependent kinase (CDK), casein kinase (CK), discoidin domain receptor (DDR), epidermal growth factor receptors (EGFR), focal adhesion kinase (FAK), Flt-3, FYN, glycogen synthase kinase (GSK), HCK, histone deacetylase (HDAC), IKK such as IKKβε, isocitrate dehydrogenase (IDH) such as IDH1, Janus kinase (JAK), KDR, lymphocyte-specific protein tyrosine kinase (LCK), lysyl oxidase protein, lysyl oxidase-like protein (LOXL), LYN, matrix metalloprotease (MMP), MEK, mitogen-activated protein kinase (MAPK), NEK9, NPM-ALK, p38 kinase, platelet-derived growth factor (PDGF), phosphorylase kinase (PK), polo-like kinase (PLK), phosphatidylinositol 3-kinase (PI3K), protein kinase (PK) such as protein kinase A, B, and/or C, PYK, spleen tyrosine kinase (SYK), serine/threonine kinase TPL2, serine/threonine kinase STK, signal transduction and transcription (STAT), SRC, serine/threonine-protein kinase (TBK) such as TBK1, TIE, tyrosine kinase (TK), vascular endothelial growth factor receptor (VEGFR), YES, or any combination thereof.

### Lymphoma or Leukemia Combination Therapy

Some chemotherapy agents are suitable for treating lymphoma or leukemia. These agents include aldesleukin, alvocidib, antineoplaston AS2-1, antineoplaston A10, anti-thymocyte globulin, amifostine trihydrate, aminocamptothecin, arsenic trioxide, beta alethine, Bcl-2 family protein inhibitor ABT-263, ABT-199, BMS-345541, bortezomib (VELCADE®), carfilzomib (Kyprolis®), vemurafenib (Zelboraf®), Omr-IgG-am (WNIG, Omrix), bryostatin 1, busulfan, carboplatin, campath-1H, CC-5103, carmustine, caspofungin acetate, clofarabine, cisplatin, cladribine, chlorambucil, curcumin, cyclosporine, cyclophosphamide, cytarabine, denileukin diftitox, dexamethasone, DT-PACE (dexamethasone, thalidomide, cisplatin, doxorubicin, cyclophosphamide, and etoposide), docetaxel, dolastatin 10, doxorubicin, doxorubicin hydrochloride, enzastaurin, epoetin alfa, etoposide, everolimus (RAD001), fenretinide, filgrastim, melphalan, mesna, flavopiridol, fludarabine, geldanamycin (17-AAG), ifosfamide, irinotecan hydrochloride, ixabepilone, lenalidomide (REVLIMID®, CC-5013), lymphokine-activated killer cells, melphalan, methotrexate, mitoxantrone hydrochloride, motexafin gadolinium, mycophenolate mofetil, nelarabine, oblimersen, obatoclax (GX15-070), oblimersen, octreotide acetate, omega-3 fatty acids, oxaliplatin, paclitaxel, PD0332991, PEGylated liposomal doxorubicin hydrochloride, pegfilgrastim, pentostatin, perifosine, prednisolone, prednisone, R-roscovitine (seliciclib, CYC202), recombinant interferon alfa, recombinant interleukin-12, recombinant interleukin-11, recombinant flt3 ligand, recombinant human thrombopoietin, rituximab, sargramostim, sildenafil citrate, simvastatin, sirolimus, styryl sulphones, tacrolimus, tanespimycin, temsirolimus (CCl-779), thalidomide, therapeutic allogeneic lymphocytes, thiotepa, tipifarnib, bortezomib (VELCADE®, PS-341), vincristine, vincristine sulfate, vinorelbine ditartrate, SAHA (suberanilohydroxamic acid, or suberoyl, anilide, and hydroxamic acid), FR (fludarabine and rituximab), CHOP (cyclophosphamide, doxorubicin, vincristine, and prednisone), CVP (cyclophosphamide, vincristine, and prednisone), FCM (fludarabine, cyclophosphamide, and mitoxantrone), FCR (fludarabine, cyclophosphamide, and rituximab), hyperCVAD (hyperfractionated cyclophosphamide, vincristine, doxorubicin, dexamethasone, methotrexate, and cytarabine), ICE (iphosphamide, carboplatin, and etoposide), MCP (mitoxantrone, chlorambucil, and prednisolone), R-CHOP (rituximab and CHOP), R-CVP (rituximab and CVP), R-FCM (rituximab and FCM), R-ICE (rituximab and ICE), and R-MCP (rituximab and MCP).

One modified approach is radioimmunotherapy, wherein a monoclonal antibody is combined with a radioisotope particle, such as indium-111, yttrium-90, and iodine-131. Examples of combination therapies include, but are not limited to, iodine-131 tositumomab (BEXXAR®), yttrium-90 ibritumomab tiuxetan (ZEVALIN®), and BEXXAR® with CHOP.

The abovementioned therapies can be supplemented or combined with stem cell transplantation or treatment. Therapeutic procedures include peripheral blood stem cell transplantation, autologous hematopoietic stem cell transplantation, autologous bone marrow transplantation, antibody therapy, biological therapy, enzyme inhibitor therapy, total body irradiation, infusion of stem cells, bone marrow ablation with stem cell support, *in vitro*-treated peripheral blood stem cell transplantation, umbilical cord blood transplantation, immunoenzyme technique, low-LET cobalt-60 gamma ray therapy, bleomycin, conventional surgery, radiation therapy, and nonmyeloablative allogeneic hematopoietic stem cell transplantation.

### Non-Hodgkin's Lymphomas Combination Therapy

Treatment of non-Hodgkin's lymphomas (NHL), especially those of B cell origin, includes using monoclonal antibodies, standard chemotherapy approaches (*e.g.,* CHOP, CVP, FCM, MCP, and the like), radioimmunotherapy, and combinations thereof, especially integration of an antibody therapy with chemotherapy.

Examples of unconjugated monoclonal antibodies for the treatment of NHL/B-cell cancers include rituximab, alemtuzumab, human or humanized anti-CD20 antibodies, lumiliximab, anti-TNF-related apoptosis-inducing ligand (anti-TRAIL), bevacizumab, galiximab, epratuzumab, SGN-40, and anti-CD74.

Examples of experimental antibody agents used in treatment of NHL/B-cell cancers include ofatumumab, ha20, PRO131921, alemtuzumab, galiximab, SGN-40, CHIR-12.12, epratuzumab, lumiliximab, apolizumab, milatuzumab, and bevacizumab.

Examples of standard regimens of chemotherapy for NHL/B-cell cancers include CHOP, FCM, CVP, MCP, R-CHOP, R-FCM, R-CVP, and R-MCP.

Examples of radioimmunotherapy for NHL/B-cell cancers include yttrium-90 ibritumomab tiuxetan (ZEVALIN®) and iodine-131 tositumomab (BEXXAR®).

### Mantle Cell Lymphoma Combination Therapy

Therapeutic treatments for mantle cell lymphoma (MCL) include combination chemotherapies such as CHOP, hyperCVAD, and FCM. These regimens can also be supplemented with the monoclonal antibody rituximab to form combination therapies R-CHOP, hyperCVAD-R, and R-FCM. Any of the abovementioned therapies may be combined with stem cell transplantation or ICE in order to treat MCL.

An alternative approach to treating MCL is immunotherapy. One immunotherapy uses monoclonal antibodies like rituximab. Another uses cancer vaccines, such as GTOP-99, which are based on the genetic makeup of an individual patient's tumor.

A modified approach to treat MCL is radioimmunotherapy, wherein a monoclonal antibody is combined with a radioisotope particle, such as iodine-131 tositumomab (BEXXAR®) and yttrium-90 ibritumomab tiuxetan (ZEVALIN®). In another example, BEXXAR® is used in sequential treatment with CHOP.Other approaches to treating MCL include autologous stem cell transplantation coupled with high-dose chemotherapy, administering proteasome inhibitors such as bortezomib (VELCADE® or PS-341), or administering antiangiogenesis agents such as thalidomide, especially in combination with rituximab.

Another treatment approach is administering drugs that lead to the degradation of Bcl-2 protein and increase cancer cell sensitivity to chemotherapy, such as oblimersen, in combination with other chemotherapeutic agents. A further treatment approach includes administering mTOR inhibitors, which can lead to inhibition of cell growth and even cell death. Non-limiting examples are sirolimus, temsirolimus (TORISEL®, CCI-779), CC-115, CC-223, SF-1126, PQR-309, voxtalisib, GSK-2126458, and temsirolimus in combination with RITUXAN®, VELCADE®, or other chemotherapeutic agents.

Other recent therapies for MCL have been disclosed. Such examples include flavopiridol, PD0332991, R-roscovitine (selicicilib, CYC202), styryl sulphones, obatoclax (GX15-070), TRAIL, Anti-TRAIL death receptors DR4 and DR5 antibodies, temsirolimus (TORISEL®, CCI-779), everolimus (RAD001), BMS-345541, curcumin, SAHA, thalidomide, lenalidomide (REVLIMID®, CC-5013), and geldanamycin (17-AAG).

### Waldenstrom's Macroglobulinemia Combination Therapy

Therapeutic agents used to treat Waldenstrom's Macroglobulinemia (WM) include perifosine, bortezomib (VELCADE®), rituximab, sildenafil citrate (VIAGRA®), CC-5103, thalidomide, epratuzumab (hLL2- anti-CD22 humanized antibody), simvastatin, enzastaurin, campath-1H, dexamethasone, DT-PACE, oblimersen, antineoplaston A10, antineoplaston AS2-1, alemtuzumab, beta alethine, cyclophosphamide, doxorubicin hydrochloride, prednisone, vincristine sulfate, fludarabine, filgrastim, melphalan, recombinant interferon alfa, carmustine, cisplatin, cyclophosphamide, cytarabine, etoposide, melphalan, dolastatin 10, indium-111 monoclonal antibody MN-14, yttrium-90 humanized epratuzumab, anti-thymocyte globulin, busulfan, cyclosporine, methotrexate, mycophenolate mofetil, therapeutic allogeneic lymphocytes, yttrium-90 ibritumomab tiuxetan, sirolimus, tacrolimus, carboplatin, thiotepa, paclitaxel, aldesleukin, docetaxel, ifosfamide, mesna, recombinant interleukin-11, recombinant interleukin-12, Bcl-2 family protein inhibitor ABT-263, denileukin diftitox, tanespimycin, everolimus, pegfilgrastim, vorinostat, alvocidib, recombinant flt3 ligand, recombinant human thrombopoietin, lymphokine-activated killer cells, amifostine trihydrate, aminocamptothecin, irinotecan hydrochloride, caspofungin acetate, clofarabine, epoetin alfa, nelarabine, pentostatin, sargramostim, vinorelbine ditartrate, WT-1 analog peptide vaccine, WT1 126-134 peptide vaccine, fenretinide, ixabepilone, oxaliplatin, monoclonal antibody CD19 (such as tisagenlecleucel-T, CART-19, CTL-019), monoclonal antibody CD20, omega-3 fatty acids, mitoxantrone hydrochloride, octreotide acetate, tositumomab, iodine-131 tositumomab, motexafin gadolinium, arsenic trioxide, tipifarnib, autologous human tumor-derived HSPPC-96, veltuzumab, bryostatin 1, PEGylated liposomal doxorubicin hydrochloride, and any combination thereof.

Examples of therapeutic procedures used to treat WM include peripheral blood stem cell transplantation, autologous hematopoietic stem cell transplantation, autologous bone marrow transplantation, antibody therapy, biological therapy, enzyme inhibitor therapy, total body irradiation, infusion of stem cells, bone marrow ablation with stem cell support, *in vitro*-treated peripheral blood stem cell transplantation, umbilical cord blood transplantation, immunoenzyme techniques, low-LET cobalt-60 gamma ray therapy, bleomycin, conventional surgery, radiation therapy, and nonmyeloablative allogeneic hematopoietic stem cell transplantation.

### Diffuse Large B-cell Lymphoma Combination Therapy

Therapeutic agents used to treat diffuse large B-cell lymphoma (DLBCL) include cyclophosphamide, doxorubicin, vincristine, prednisone, anti-CD20 monoclonal antibodies, etoposide, bleomycin, many of the agents listed for WM, and any combination thereof, such as ICE and R-ICE.

### Chronic Lymphocytic Leukemia Combination Therapy

Examples of therapeutic agents used to treat chronic lymphocytic leukemia (CLL) include chlorambucil, cyclophosphamide, fludarabine, pentostatin, cladribine, doxorubicin, vincristine, prednisone, prednisolone, alemtuzumab, many of the agents listed for WM, and combination chemotherapy and chemoimmunotherapy, including the following common combination regimens: CVP, R-CVP, ICE, R-ICE, FCR, and FR.

### Myelofibrosis Combination Therapy

Myelofibrosis inhibiting agents include, but are not limited to, hedgehog inhibitors, histone deacetylase (HDAC) inhibitors, and tyrosine kinase inhibitors. A non-limiting example of hedgehog inhibitors is saridegib.Examples of HDAC inhibitors include, but are not limited to, pracinostat and panobinostat.Non-limiting examples of tyrosine kinase inhibitors are lestaurtinib, bosutinib, imatinib, gilteritinib, radotinib, and cabozantinib.

Hyperproliferative Disorder Combination Therapy. Gemcitabine, nab-paclitaxel, and gemcitabine/nab-paclitaxel may be used with a JAK inhibitor and/or PI3Kδ inhibitor to treat hyperproliferative disorders.

### Kinase Inhibitors

In one embodiment, the compound described herein may be used or combined with one or more additional therapeutic agents. The one or more therapeutic agents include, but are not limited to, an inhibitor of Abl, activated CDC kinase (ACK) such as ACK1, adenosine A2B receptor (A2B), apoptosis signal-regulating kinase (ASK), Aurora kinase, Bruton's tyrosine kinase (BTK), BET-bromodomain (BRD) such as BRD4, c-Kit, c-Met, CDK-activating kinase (CAK), calmodulin-dependent protein kinase (CaMK), cyclin-dependent kinase (CDK), casein kinase (CK), discoidin domain receptor (DDR), epidermal growth factor receptors (EGFR), focal adhesion kinase (FAK), Flt-3, farnesoid x receptor (FXR), FYN, glycogen synthase kinase (GSK), HCK, histone deacetylase (HDAC), indoleamine 2,3-dioxygenase (IDO), I-Kappa-B kinase (IKK) such as IKKβε, isocitrate dehydrogenase (IDH) such as IDH1, Janus kinase (JAK), KDR, lysine demethylase (KDM5), lymphocyte-specific protein tyrosine kinase (LCK), lysyl oxidase protein (LOX), lysyl oxidase-like protein (LOXL), LYN, matrix metalloprotease (MMP), mitogen-activated protein kinase (MEK), mitogen-activated protein kinase (MAPK), mut T homolog (MTH), NEK9, NPM-ALK, p38 kinase, platelet-derived growth factor (PDGF), phosphorylase kinase (PK), polo-like kinase (PLK), phosphatidylinositol 3-kinase (PI3K), protein kinase (PK) such as protein kinase A, B, and/or C, PYK, spleen tyrosine kinase (SYK), serine/threonine kinase TPL2, serine/threonine kinase (STK), signal transduction and transcription (STAT), SRC, serine/threonine-protein kinase (TBK) such as TBK1, TIE, tyrosine kinase (TK), tank-binding kinase (TBK), vascular endothelial growth factor receptor (VEGFR), YES, or any combination thereof.

### Apoptosis Signal-Regulating Kinase (ASK) Inhibitors

ASK inhibitors include ASK1 inhibitors. Examples of ASK1 inhibitors include, but are not limited to, those described in WO 2011/008709 (Gilead Sciences) and WO 2013/112741 (Gilead Sciences).

### Mitogen-activated Protein Kinase (MEK) Inhibitors

MEK inhibitors include selumetinib, MT-144, sorafenib, trametinib (GSK1120212), binimetinib, antroquinonol, uprosertib + trametinib.

### Casein Kinase (CK) Inhibitors

CK inhibitors include CK1 and/or CK2.

### Cyclin-dependent Kinase (CDK) Inhibitors

CDK inhibitors include inhibitors of CDK 1, 2, 3, 4, and/or 6. Examples of CDK inhibitors include rigosertib, selinexor, UCN-01, alvocidib (HMR-1275, flavopiridol), FLX-925, AT-7519, abemaciclib, palbociclib, and TG-02. Discoidin Domain Receptor (DDR) Inhibitors

DDR inhibitors include inhibitors of DDR1 and/or DDR2. Examples of DDR inhibitors include, but are not limited to, those disclosed in WO 2014/047624 (Gilead Sciences), US 2009-0142345 (Takeda Pharmaceutical), US 2011-0287011 (Oncomed Pharmaceuticals), WO 2013/027802 (Chugai Pharmaceutical), and WO 2013/034933 (Imperial Innovations).

### Histone Deacetylase (HDAC) Inhibitors

Examples of HDAC inhibitors include, but are not limited to, pracinostat, CS-055 (HBI-8000), resminostat, entinostat, abexinostat, belinostat, vorinostat, riclinostat, CUDC-907, ACY-241, CKD-581, SHP-141, valproic acid (VAL-001), givinostat, quisinostat (JNJ-26481585), BEBT-908 and panobinostat. Janus Kinase (JAK) Inhibitors

JAK inhibitors inhibit JAK1, JAK2, and/or JAK3. Examples of JAK inhibitors include, but are not limited to, Compound A, momelotinib (CYT0387), ruxolitinib, filgotinib (GLPG0634), peficitinib (ASP015K), fedratinib, tofacitinib, baricitinib, lestaurtinib, pacritinib, XL019, AZD1480, INCB039110, LY2784544, BMS911543, and NS018.

### Lysyl Oxidase-Like Protein (LOXL) Inhibitors

LOXL inhibitors include inhibitors of LOXL1, LOXL2, LOXL3, LOXL4, and/or LOXL5. Examples of LOXL inhibitors include, but are not limited to, the antibodies described in WO 2009/017833 (Arresto Biosciences). Examples of LOXL2 inhibitors include, but are not limited to, the antibodies described in WO 2009/017833 (Arresto Biosciences), WO 2009/035791 (Arresto Biosciences), and WO 2011/097513 (Gilead Biologics).

### Matrix Metalloprotease (MMP) Inhibitors

MMP inhibitors include inhibitors of MMP1 through 10. Examples of MMP9 inhibitors include, but are not limited to, marimastat (BB-2516), cipemastat (Ro 32-3555), and those described in WO 2012/027721 (Gilead Biologics).

### Polo-like Kinase (PLK) Inhibitors

PLK inhibitors include inhibitors of PLK 1, 2, and 3.

### Phosphatidylinositol 3-kinase (PI3K) Inhibitors

PI3K inhibitors include inhibitors of PI3Kγ, PI3Kδ, PI3Kβ, PI3Kα, and/or pan-PI3K. Examples of PI3K inhibitors include, but are not limited to, wortmannin, BKM120, CH5132799, XL756, idelalisib (Zydelig®), and GDC-0980. Examples of PI3Kγ inhibitors include, but are not limited to, ZSTK474, AS252424, LY294002, and TGI00115.Examples of PI3Kδ inhibitors include, but are not limited to, Compound B, Compound C, Compound D, Compound E, PI3K II, TGR-1202, AMG-319, GSK2269557, X-339, X-414, RP5090, KAR4141, XL499, OXY111A, IPI-145, IPI-443, and the compounds described in WO 2005/113556 (ICOS), WO 2013/052699 (Gilead Calistoga), WO 2013/116562 (Gilead Calistoga), WO 2014/100765 (Gilead Calistoga), WO 2014/100767 (Gilead Calistoga), and WO 2014/201409 (Gilead Sciences). Examples of P13Kβ inhibitors include, but are not limited to, GSK2636771, BAY 10824391, and TGX221. Examples of PI3Kα inhibitors include, but are not limited to, buparlisib, BAY 80-6946, BYL719, PX-866, RG7604, MLN1117, WX-037, AEZA-129, and PA799.Examples of pan-PI3K inhibitors include, but are not limited to, LY294002, BEZ235, XL147 (SAR245408), and GDC-0941. Spleen Tyrosine Kinase (SYK) Inhibitors

Examples of SYK inhibitors include, but are not limited to, 6-(1H-indazol-6-yl)-N-(4-morpholinophenyl)imidazo[1,2-a]pyrazin-8-amine, tamatinib (R406), fostamatinib (R788), PRT062607, BAY-61-3606, NVP-QAB 205 AA, R112, R343, and those described in US 8450321 (Gilead Connecticut). Tyrosine-kinase Inhibitors (TKIs)

TKIs may target epidermal growth factor receptors (EGFRs) and receptors for fibroblast growth factor (FGF), platelet-derived growth factor (PDGF), and vascular endothelial growth factor (VEGF). Examples of TKIs that target EGFR include, but are not limited to, gefitinib, nintedanib, and erlotinib. Sunitinib is a non-limiting example of a TKI that targets receptors for FGF, PDGF, and VEGF. Additional TKIs include dasatinib, ponatinib.

### Toll-like Receptor (TLR) Modulators

TLR modulators include inhibitors of TLR-1, TLR-2, TLR-3, TLR-4, TLR-5, TLR-6, TLR-7, TLR-8, TLR-9, TLR-10, TLR-11, TLR-12, and/or TLR-13.

### Articles of Manufacture and Kits

Compositions (including, for example, formulations and unit dosages) comprising a Btk inhibitor, as described herein, and compositions comprising a checkpoint inhibitor, as described herein, can be prepared and placed in an appropriate container, and labeled for treatment of an indicated condition. Accordingly, provided is also an article of manufacture for use in treating cancer in a human in need thereof, wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab;
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

The article, such as a container, comprises (i) a unit dosage form of a Btk inhibitor and a unit dosage form of a checkpoint inhibitor, as described herein, and a label containing instructions for use of the compounds. In some embodiments, the article of manufacture is a container comprising (i) a unit dosage form of a Btk inhibitor, as described herein, and one or more pharmaceutically acceptable carriers, adjuvants or excipients; and (ii) a unit dosage form of a checkpoint inhibitor, as described herein, and one or more pharmaceutically acceptable carriers, adjuvants or excipients. In one embodiment, the unit dosage form for both the Btk inhibitor and the checkpoint inhibitor is a tablet.

Kits also are contemplated for use according to the invention. Accordingly, provided is also a kit for use in treating cancer in a human in need thereof, the kit comprising (i) a pharmaceutical composition comprising a Btk inhibitor, as described herein, and (ii) a pharmaceutical composition comprising a checkpoint inhibitor, as described herein, wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

The kit may include a package insert containing instructions for use of the composition in treatment of a medical condition. In some embodiments, the kit comprises (i) a unit dosage form of the Btk inhibitor, as described herein, and one or more pharmaceutically acceptable carriers, adjuvants or excipients; and (ii) a unit dosage form of a checkpoint inhibitor, as described herein, and one or more pharmaceutically acceptable carriers, adjuvants or excipients. In one embodiment, the unit dosage form for both the Btk inhibitor and the checkpoint inhibitor is a tablet.

The instructions for use in the kit are for treating a cancer, including, for example, a hematologic malignancy or a solid tumor, as further described herein.

The Btk inhibitor, Compound A1, itself, as well as the combinations of Compound A1 and a protein checkpoint inhibiting compound discussed herein, may be used or combined with a chemotherapeutic agent, an anti-cancer agent, an anti-angiogenic agent, an anti-fibrotic agent, an immunotherapeutic agent, a therapeutic antibody, a radiotherapeutic agent, an anti-neoplastic agent, an anti-proliferation agent, or any combination thereof. These therapeutic agents may be in the forms of compounds, antibodies, polypeptides, or polynucleotides. In one embodiment, the application provides a product comprising a pharmaceutically effective amount of Compound A1, or a pharmaceutically acceptable salt or hydrate thereof, a pharmaceutically effective amount of a checkpoint protein inhibiting compound wherein the checkpoint inhibitor is an inhibitor of PD-1, and a pharmaceutically effective amount of an additional therapeutic agent selected from the groups discussed below, as a combined preparation for simultaneous, separate, or sequential use in treating a cancer, such as hematological cancers and solid tumors, in a human in need thereof.

The compound described herein may be used or combined with one or more of the following additional therapeutic agents: an adenosine A2B receptor (A2B) inhibitor, a BET-bromodomain 4 (BRD4) inhibitor, an isocitrate dehydrogenase 1 (IDH1) inhibitor, an IKK inhibitor, a protein kinase C (PKC) activator or inhibitor, a TPL2 inhibitor, a serine/threonine-protein kinase 1 (TBK1) inhibitor, agents that activate or reactivate latent human immunodeficiency virus (HIV) such as panobinostat or romidepsin, an anti-CD20 antibody such as rituximab, ofatuzumab, obinutuzumab, an anti-programmed cell death protein 1 (anti-PD-1) antibody such as nivolumab (BMS-936558, or MDX1106, or MK-34775), and pembrolizumab (MK-3475, SCH-900475, lambrolizumab), and anti-programmed death-ligand 1 (anti-PD-Ll) antibodies such as BMS-936559 (MDX1105), atezolizumab (MPDL3280A), durvalumab (MEDI-4736), and avelumab (MSB0010718C).

The combination of a Btk inhibitor and a checkpoint inhibitor as disclosed herein, and the one or more therapeutic agents (*e.g.* an A2B inhibitor, an apoptosis signal-regulating kinase (ASK) inhibitor, a BRD4 inhibitor, a discoidin domain receptor 1 (DDR1) inhibitor, a histone deacetylase (HDAC) inhibitor, an isocitrate dehydrogenase (IDH) inhibitor, a Janus kinase (JAK) inhibitor, a lysyl oxidase-like protein 2 (LOXL2) inhibitor, a matrix metalloprotease 9 (MMP9) inhibitor, a phosphatidylinositol 3-kinase (PI3K) inhibitor, a PKC activator or inhibitor, a spleen tyrosine kinase (SYK) inhibitor, a TPL2 inhibitor, or a TBK inhibitor) may be further used or combined with a chemotherapeutic agent, an anti-cancer agent, an anti-angiogenic agent, an anti-fibrotic agent, an immunotherapeutic agent, a therapeutic antibody, a radiotherapeutic agent, an anti-neoplastic agent, a smoothened (SMO) receptor inhibitor, or any combination thereof.

### EXAMPLES

The following reference examples are provided to further aid in understanding the embodiments disclosed in the application, and presuppose an understanding of conventional methods well known to those persons having ordinary skill in the art to which the examples pertain. The particular materials and conditions described hereunder are intended to exemplify particular aspects of embodiments disclosed herein. It is understood that the conditions (such as the reagent concentration or the incubation temperature) of the assay or study may be varied and the results of the assay or study may vary. In some instances, the value may vary within a range of one to three-fold.

### Reference Example 1

This example evaluated in vivo anti-tumor activity of a BTK inhibitor Compound A1 in combination with an anti-PD-1 antibody (4H2), (Kitayama Labes Co., Ltd) in syngeneic tumors.

A20 cell line (ATCC), mouse B-cell lymphoma, were maintained in lymphocyte growth medium (LGM): RPMI-1640 supplemented with 10% FBS, 1% penicillin-streptomycin. Fresh 1% (vol) Monothioglycerol was also added to the culture. The cell suspension of 2 × 10⁶ to 4 × 10⁶ cells per dish was cultured at 37°C, 5% CO₂. The cells were suspended in PBS (5 × 10⁷ cells/mL).

A20-bearing BALB/c mice were subcutaneously injected (5 × 10⁶ cells per animal). Day 6 after cell injection (day 0), mice having the tumor volume of about 200 mm³ were randomized into four groups (n = 9 per group) for treatment with vehicle (CRF-1), Compound A1, 4H2, or Compound A1 and 4H2. Mice were fed diets containing of Compound A1. 4H2 was administered intraperitoneally at 20 mg/kg on day 0 followed by 10 mg/kg every 6 days; the average dose of Compound A1 was 46 mg/kg/day (CRF-1 at 0.037%) in the first and second studies or 15 mg/kg/day (CRF-1 at 0.012%) in the second study . During the studies, tumor volume (long axis of tumor × (short axis of tumor)² × 0.5), tumor growth inhibition rate (TGI, as compared to tumors of the vehicle control group), temporal changes in tumor volume, body weight, or food intake were determined. The studies were terminated when tumors reached a maximum of 3,000 mm³.

In the first study, the effects of Compound A1 at 46 mg/kg/day were evaluated. The first study was terminated at day 19. For the group treated with Compound A1 only, at day 0 TGI was 0.8%; at day 2 TGI was 14%; at day 6 TGI was 23%; at day 9 TGI was -12%; at day 12 TGI was 9%; at day 15 TGI was 8%; and at day 19 TGI was 10%. For the group treated with 4H2 only, at day 0 TGI was 0.2%; at day 2 TGI was 21%; at day 6 TGI was 24%; at day 9 TGI was 26%; at day 12 TGI was 43%; at day 15 TGI was 51%; and at day 19 TGI was 55%. For the group treated with 4H2 and Compound A1, at day 0 TGI was 0%; at day 2 TGI was 17%; at day 6 TGI was 41%; at day 9 TGI was 47%; at day 12 TGI was 53%; at day 15 TGI was 63%; and at day 19 TGI was 64%.

The average tumor volume (mm³) (± standard error) for the control group at day 0 was 170 (±9); day 2 was 288 (±21); day 6 was 429 (±55); day 9 was 564 (±82); day 12 was 882 (±153); day 15 was 1536 (±247); and day 19 was 2822 (±432). Tumor volume in mm³ for the group treated with Compound A1 only at day 0 was 168 (±11); day 2 was 248 (±18); day 6 was 331 (±32); day 9 was 632 (±61); day 12 was 805 (±104); day 15 was 1408 (±157); and day 19 was 2553 (±272). Tumor volume in mm³ for the group treated with 4H2 only at day 0 was 169 (±10); day 2 was 226 (±11); day 6 was 325 (±22); day 9 was 417 (±38); day 12 was 506 (±88); day 15 was 751 (±152); and day 19 was 1282 (±318). Tumor volume in mm³ for the group treated with 4H2 and Compound A1 at day 0 was 170 (±10); day 2 was 238 (±12); day 6 was 254 (±29); day 9 was 302 (±67); day 12 was 418 (±126); day 15 was 567 (±211); and day 19 was 1007 (±377).

In addition, body weight (g) was measured over 19 days. Average body weight (g) (± standard error) for the control group at day 0 was 19 (±0.5); day 2 was 20 (±0.5); day 6 was 20 (±0.5); day 9 was 20 (±0.4); day 12 was 21 (±0.4); day 15 was 22 (±0.4); and day 19 was 22 (±0.7). Average body weight (g) (± standard error) for the group treated with Compound A1 only at day 0 was 19 (±0.4); day 2 was 19 (±0.4); day 6 was 20 (±0.3); day 9 was 20 (±0.3); day 12 was 21 (±0.3); day 15 was 21 (±0.3); and day 19 was 22 (±0.4). Average body weight (g) (± standard error) for the group treated with 4H2 only at day 0 was 20 (±0.4); day 2 was 20 (±0.4); day 6 was 20 (±0.4); day 9 was 20 (±0.4); day 12 was 21 (±0.4); day 15 was 21 (±0.4); and day 19 was 21 (±0.5). Average body weight (g) (± standard error) for the group treated with 4H2 and Compound A1 at day 0 was 19 (±0.2); day 2 was 20 (±0.3); day 6 was 20 (±0.3); day 9 was 20 (±0.3); day 12 was 21 (±0.3); day 15 was 21 (±0.3); and day 19 was 22 (±0.4).

On day 19, tumor remission was observed in 3 out of 9 mice treated with Compound A1 and 4H2. No tumor remission was observed in other groups.

In the second study, the effects of Compound A1 at 15 mg/kg/day (CRF-1 at 0.012%) and 46 mg/kg/day (CRF-1 at 0.037%) were evaluated. Similar protocols as described above were used. The study (n = 12 per group) was terminated at day 17. No results were presented to the group treated with 4H2 only at day 17; one mice in the group exhibited tumor volume higher than 3,000 mm³ at day 14.

For the group treated with 4H2 only, at day 0 TGI was 0.4%; at day 3 TGI was 0.2%; at day 7 TGI was 12%; at day 10 TGI was 29%; and at day 14 TGI was 37%. For the group treated with 4H2 and Compound A1 at 0.012%, at day 0 TGI was 0.9%; at day 3 TGI was 18%; at day 7 TGI was 20%; at day 10 TGI was 40%; at day 14 TGI was 41%; and at day 17 at 36%. For the group treated with 4H2 and Compound A1 at 0.037%, at day 0 TGI was 0.1%; at day 3 TGI was 3%; at day 7 TGI was 4%; at day 10 TGI was 10%; at day 14 TGI was 16%; and at day 17 TGI was 19%.

The average tumor volume (± standard error) for the control group at day 0 was 200 (±10); day 3 was 281 (±19); day 7 was 536 (±53); day 10 was 964 (±115); day 14 was 1707 (±201); and day 17 was 2999 (±335). Tumor volume in mm³ for the group treated with 4H2 only at day 0 was 199 (±11); day 3 was 280 (±27); day 7 was 474 (±104); day 10 was 684 (±165); and day 14 was 1074 (±300). Tumor volume in mm³ for the group treated with 4H2 and Compound A1 at 0.012% at day 0 was 198 (±9); day 3 was 231 (±21); day 7 was 427 (±89); day 10 was 577 (±144); day 14 was 1002 (±290); and day 17 was 1912 (±610). Tumor volume in mm³ for the group treated with 4H2 and Compound A1 at 0.037% at day 0 was 200 (±9); day 3 was 273 (±25); day 7 was 517 (±82); day 10 was 868 (±170); day 14 was 1432 (±311); and day 17 was 2440 (±526).

The average body weight (± standard error) for control group at day 0 was 19 (±0.2); day 3 was 19 (±0.3); day 7 was 20 (±0.3); day 10 was 20 (±0.3); day 14 was 21 (±0.3), and day 17 was 22 (±0.4). The average body weight (± standard error) for the group treated with 4H2 only at day 0 was 20 (±0.2); day 3 was 20 (±0.2); day 7 was 20 (±0.2); day 10 was 21 (±0.2); and day 14 was 21 (±0.4). Average body weight (g) (± standard error) for the group treated with 4H2 and Compound A1 at 0.012% at day 0 was 19 (±0.3); day 3 was 20 (±0.3); day 7 was 20 (±0.3); day 10 was 20 (±0.2); day 14 was 21 (±0.3); and day 17 was 22 (±0.4). Average body weight (g) (± standard error) for the group treated with 4H2 and Compound A1 at 0.037% at day 0 was 19 (±0.5); day 3 was 19 (±0.5); day 7 was 20 (±0.4); day 10 was 20 (±0.5); day 14 was 21 (±0.5); and day 17 was 23 (±0.6).

At day 17, tumor remission was observed in four mice in the group treated with Compound A1 (0.012%) and 4H2, two mice in the group treated with Compound A1 (0.037%) and 4H2. No tumor remission was observed in other groups.

### Reference Example 2

This example evaluated in vivo anti-tumor activity of a BTK inhibitor (Compound A1) in combination with an anti-PD-1 antibody (4H2) in syngeneic tumors. Similar protocols as described above were used with L1210 cell line (JCRB), mouse lymphocytic leukemia.

L1210-bearing DBA-2 mice were subcutaneously injected (5 × 10⁵ cells/animal). Mice were randomized into four groups (n = 8 per group) for treatment with vehicle (CRF-1), Compound A1, 4H2 (10 mg/kg every 6 days), or Compound A1 and 4H2. Mice were fed diets containing of Compound A1. 4H2 was administered intraperitoneally at 20 mg/kg on day 0 followed by 10 mg/kg every 6 days. The average dose of Compound A1 was 54 mg/kg/day (CRF-1 at 0.037%).

The study was terminated at day 14. For the group treated with Compound A1 only, at day 4 TGI was 8%; at day 7 TGI was 30%; at day 10 TGI was 7%; at day 12 TGI was -0.7%; and day 14 at -0.2%. For the group treated with 4H2 only at day 4 TGI was -5%; at day 7 TGI was 7%; day 10 TGI was 5%; at day 12 TGI was 13%; and day 14 at 19%. For the group treated with 4H2 and Compound A1, at day 4 TGI was -23%; at day 7 TGI was 19%; day 10 TGI was 22%; at day 12 TGI was 42%; and day 14 TGI was 46%.

The average tumor volume (± standard error) for the control group at day 0 was 0 (±0); day 4 was 78 (±12); day 7 was 354 (±20); day 10 was 942 (±39); day 12 was 1838 (±106); and day 14 was 3273 (±224). Tumor volume in mm³ for the group treated with Compound A1 only at day 0 was 0 (±0); day 4 was 71 (±16); day 7 was 247 (±34); day 10 was 874 (±108); day 12 was 1851 (±226); and day 14 was 3279 (±321). Tumor volume in mm³ for the group treated with 4H2 only at day 0 was 0 (±0); day 4 was 82 (±14); day 7 was 329 (±41); day 10 was 892 (±116); day 12 was 1598 (±184); and day 14 was 2656 (±294). Tumor volume in mm³ for the group treated with 4H2 and Compound A1 at day 0 was 0 (±0); day 4 was 96 (±12); day 7 was 286 (±26); day 10 was 734 (±52); day 12 was 1068 (±73); and day 14 was 1774 (±159).

In addition, average body weight (g) (± standard error) for the control group at day 0 was 15 (±0.4); day 4 was 15 (±0.4); day 7 was 15 (±0.5); day 10 was 16 (±0.5); day 12 was 17 (±0.4); and day 14 was 18 (±0.5). The average body weight (g) (± standard error) for the group treated with Compound A1 only at day 0 was 15 (±0.4); day 4 was 16 (±0.4); day 7 was 16 (±0.4); day 10 was 17 (±0.5); day 12 was 18 (±0.4); and day 14 was 19 (±0.5). The average body weight (g) (± standard error) for the group treated with 4H2 only at day 0 was 15 (±0.4); day 4 was 15 (±0.3); day 7 was 15 (±0.4); day 10 was 17 (±0.5); day 12 was 17 (±0.5); and day 14 was 18 (±0.6). The average body weight (g) (± standard error) for the group treated with 4H2 and Compound A1 at day 0 was 15 (±0.4); day 4 was 16 (±0.4); day 7 was 16 (±0.4); day 10 was 17 (±0.4); day 12 was 17 (±0.4); and day 14 was 18 (±0.3).

### Reference Example 3

This example evaluates the effects of BTK inhibitor in a pancreatic intraepithelial neoplasia (PanIN) orthotopic model (i.e. immuno-compromised mice comprising a xenograph of human PanIN cells). The combination of BTK inhibitor, gemcitabine and/or PD-1 or PD-L1 inhibitor may result in a tumor growth inhibition and/or remission. Tumor cells are implanted or injected into the pancreas of the mice. Two weeks after implantation or injection, the mice were randomized into five groups (n = 14 per group). During the studies, FACS or IHC analysis, tumor size/weight, biomarker analysis or the like may be evaluated for potential effects. Each group receives different treatment: a vehicle control, gemcitabine only; gemcitabine in combination with a PD-1 or PD-L1 inhibitor; gemcitabine in combination with a Btk inhibitor; and gemcitabine in combination a Btk inhibitor and a PD-1 or PD-L1 inhibitor. The Btk inhibitor may be Compound A1; the PD-1 or PD-L1 inhibitor may be an anti-PD-1 or anti-PD-Ll antibody. The study may be terminated at two, three, or four weeks after treatment.

## Claims

1. A combination of a therapeutically effective amount of a Btk inhibitor and a therapeutically effective amount of a checkpoint inhibitor for use in treating cancer in a human in need thereof,
wherein the Btk inhibitor has the chemical structure: 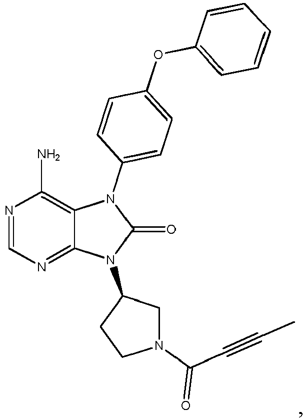 and wherein the checkpoint inhibitor is an inhibitor of PD-1; and wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

2. The combination for use according to Claim 1, wherein the Btk inhibitor and/or the checkpoint inhibitor is to be administered intravenously, intramuscularly, parenterally, nasally or orally.

3. The combination for use according to Claim 1, wherein the Btk inhibitor is to be administered prior, after or concurrently with the checkpoint inhibitor.

4. The combination for use according to Claim 1, wherein the cancer is selected from the group consisting of hematologic malignancy, leukemia, lymphoma, and multiple myeloma.

5. The combination for use according to Claim 1 wherein the inhibitor of PD-1 is an anti-PD-1 antibody selected from the group of nivolumab, and pembrolizumab.

6. An article of manufacture for use in treating cancer in a human in need thereof, the article comprising:
(i) a unit dosage form of a Btk inhibitor, wherein the Btk inhibitor is 6-amino-9-[(3R)-1-(2-butynoyl)-3-pyrrolidinyl]-7-(4-phenoxyphenyl)-7,9-dihydro-8H -purin-8-one; and
(ii) a unit dosage form of a checkpoint inhibitor, wherein the checkpoint inhibitor is an inhibitor of PD-1; and
(iii) a label containing instructions for use of the Btk inhibitor and the checkpoint inhibitor in treating cancer;
wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

7. The article of manufacture for use according to Claim 6 wherein the inhibitor of PD-1 is an anti-PD-1 antibody selected from the group of nivolumab, and pembrolizumab.

8. A kit for use in treating cancer in a human in need thereof, the kit comprising:
(i) a pharmaceutical composition comprising a Btk inhibitor, wherein the Btk inhibitor has the structure: 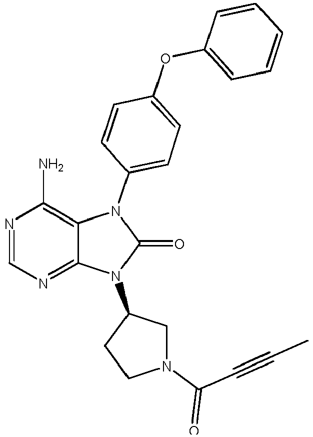
(ii) a pharmaceutical composition comprising a checkpoint inhibitor, wherein the checkpoint inhibitor is an inhibitor of PD-1;
wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

9. The kit for use according to claim 8 wherein the inhibitor of PD-1 is an anti-PD-1 antibody selected from the group of nivolumab and pembrolizumab.

10. The combination for use according to claim 1, 6 or 8, wherein the human is (i) refractory to the at least one anti-cancer therapy, and (ii) is in relapse after treatment with the at least one anti-cancer therapy.

11. A combination of a Btk inhibitor and a checkpoint inhibitor for use in sensitizing a human who is (i) refractory to at least one chemotherapy treatment, or (ii) in relapse after treatment with chemotherapy, or both (i) and (ii); wherein the Btk inhibitor has the chemical structure: 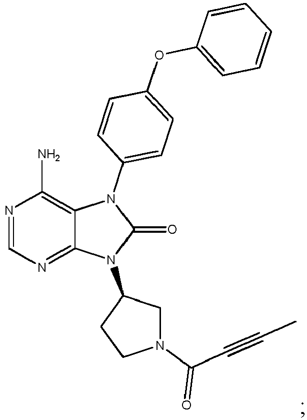 wherein the checkpoint inhibitor is an inhibitor of PD-1; and wherein the human is refractory to at least one of the cancer therapies, or is in relapse after treatment with at least one anti-cancer therapy selected from the group of:
a) fludarabine;
b) rituximab;
c) rituximab combined with fludarabine;
d) cyclophosphamide combined with fludarabine;
e) cyclophosphamide combined with rituximab and fludarabine;
f) cyclophosphamide combined with vincristine and prednisone;
g) cyclophosphamide combined with vincristine, prednisone, and rituximab;
h) a combination of cyclophosphamide, doxorubicin, vincristine, and prednisone;
i) Chlorambucil combined with prednisone, rituximab, obinutuzumab, or ofatumumab
j) pentostatin combined with cyclophosphamide and rituximab;
k) bendamustine combined with rituximab;
l) alemtuzumab;
m) fludarabine plus cyclophosphamide, bendamustine, or chlorambucil; and
n) fludarabine plus cyclophosphamide, bendamustine, or chlorambucil, combined with an anti-CD20 antibody.

12. The combination for use according to Claim 1, 6, 8 or 11, wherein the anti-CD20 antibody is selected from the group of rituximab, ofatumumab, and obinutuzumab.

## Patentansprüche

1. Kombination aus einer therapeutisch wirksamen Menge eines Btk-Inhibitors und einer therapeutisch wirksamen Menge eines Checkpoint-Inhibitors zur Verwendung bei der Behandlung von Krebs bei einem Menschen, der dessen bedarf, wobei der Btk-Inhibitor die chemische Struktur 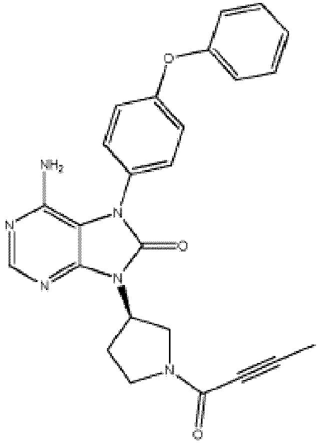 hat und wobei der Checkpoint-Inhibitor ein Inhibitor von PD-1 ist und wobei der Mensch gegenüber mindestens einer der Krebstherapien refraktär ist oder nach einer Behandlung mit mindestens einer Anti-Krebstherapie, ausgewählt aus der Gruppe
a) Fludarabin,
b) Rituximab,
c) Rituximab kombiniert mit Fludarabin,
d) Cyclophosphamid kombiniert mit Fludarabin,
e) Cyclophosphamid kombiniert mit Rituximab und Fludarabin,
f) Cyclophosphamid kombiniert mit Vincristin und Prednison,
g) Cyclophosphamid kombiniert mit Vincristin, Prednison und Rituximab,
h) eine Kombination aus Cyclophosphamid, Doxorubicin, Vincristin und Prednison,
i) Chlorambucil kombiniert mit Prednison, Rituximab, Obinutuzumab oder Ofatumumab
j) Pentostatin kombiniert mit Cyclophosphamid und Rituximab,
k) Bendamustin kombiniert mit Rituximab,
l) Alemtuzumab,
m) Fludarabin plus Cyclophosphamid, Bendamustin oder Chlorambucil, und
n) Fludarabin plus Cyclophosphamid, Bendamustin oder Chlorambucil, kombiniert mit einem Anti-CD20-Antikörper,
einen Rückfall erleidet.

2. Kombination zur Verwendung nach Anspruch 1, wobei der Btk-Inhibitor und/oder der Checkpoint-Inhibitor intravenös, intramuskulär, parenteral, nasal oder oral zu verabreichen ist.

3. Kombination zur Verwendung nach Anspruch 1, wobei der Btk-Inhibitor vor, nach oder gleichzeitig mit dem Checkpoint-Inhibitor zu verabreichen ist.

4. Kombination zur Verwendung nach Anspruch 1, wobei der Krebs ausgewählt ist aus der aus hämatologischem Malignom, Leukämie, Lymphom und multiplem Myelom bestehenden Gruppe.

5. Kombination zur Verwendung gemäß Anspruch 1, wobei der PD-1-Inhibitor ein aus der Gruppe Nivolumab und Pembrolizumab ausgewählter Anti-PD-1-Antikörper ist.

6. Erzeugnis zur Verwendung bei der Behandlung von Krebs bei einem Menschen, der dessen bedarf, wobei das Erzeugnis umfasst:
(i) eine Einzeldosis eines Btk-Inhibitors, wobei der Btk-Inhibitor 6-Amino-9-[(3R)-1-(2-butinoyl)-3-pyrrolidinyl]-7-(4-phenoxyphenyl)-7,9-dihydro-8H-purin-8-on ist, und
(ii) eine Einzeldosis eines Checkpoint-Inhibitors, wobei der Checkpoint-Inhibitor ein Inhibitor von PD-1 ist, und
(iii) ein Etikett, das Anweisungen zur Verwendung des Btk-Inhibitors und des Checkpoint-Inhibitors bei der Behandlung von Krebs enthält,
wobei der Mensch gegenüber mindestens einer der Krebstherapien refraktär ist oder nach einer Behandlung mit mindestens einer Anti-Krebstherapie, ausgewählt aus der Gruppe
a) Fludarabin,
b) Rituximab,
c) Rituximab kombiniert mit Fludarabin,
d) Cyclophosphamid kombiniert mit Fludarabin,
e) Cyclophosphamid kombiniert mit Rituximab und Fludarabin,
f) Cyclophosphamid kombiniert mit Vincristin und Prednison,
g) Cyclophosphamid kombiniert mit Vincristin, Prednison und Rituximab,
h) eine Kombination aus Cyclophosphamid, Doxorubicin, Vincristin und Prednison,
i) Chlorambucil kombiniertmit Prednison, Rituximab, Obinutuzumab oder Ofatumumab,
j) Pentostatin kombiniert mit Cyclophosphamid und Rituximab,
k) Bendamustin kombiniert mit Rituximab,
l) Alemtuzumab,
m) Fludarabin plus Cyclophosphamid, Bendamustin oder Chlorambucil und
n) Fludarabin plus Cyclophosphamid, Bendamustin oder Chlorambucil, kombiniert mit einem Anti-CD20-Antikörper,
einen Rückfall erleidet.

7. Erzeugnis zur Verwendung gemäß Anspruch 6, wobei der Inhibitor von PD-1 ein aus der Gruppe Nivolumab und Pembrolizumab ausgewählter Anti-PD-1-Antikörper ist.

8. Kit zur Verwendung bei der Behandlung von Krebs bei einem Menschen, der dessen bedarf, wobei das Kit umfasst:
(i) eine pharmazeutische Zusammensetzung mit einem Btk-Inhibitor, wobei der Btk-Inhibitor die folgende Struktur aufweist: 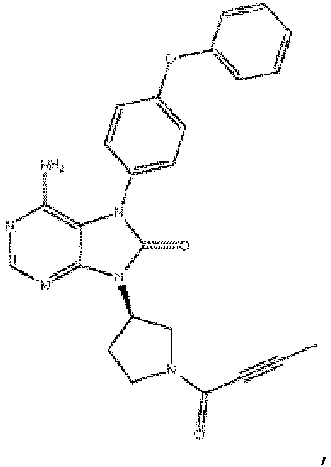
(ii) eine pharmazeutische Zusammensetzung mit einem Checkpoint-Inhibitor, wobei der Checkpoint-Inhibitor ein Inhibitor von PD-1 ist,
wobei der Mensch refraktär gegenüber mindestens einer der Krebstherapien ist oder nach der Behandlung mit mindestens einer Anti-Krebstherapie, ausgewählt aus der Gruppe
a) Fludarabin,
b) Rituximab,
c) Rituximab kombiniert mit Fludarabin,
d) Cyclophosphamid kombiniert mit Fludarabin,
e) Cyclophosphamid kombiniert mit Rituximab und Fludarabin,
f) Cyclophosphamid kombiniert mit Vincristin und Prednison,
g) Cyclophosphamid kombiniert mit Vincristin, Prednison und Rituximab,
h) eine Kombination aus Cyclophosphamid, Doxorubicin, Vincristin und Prednison,
i) Chlorambucil kombiniert mit Prednison, Rituximab, Obinutuzumab oder Ofatumumab
j) Pentostatin kombiniert mit Cyclophosphamid und Rituximab,
k) Bendamustin kombiniert mit Rituximab,
l) Alemtuzumab,
m) Fludarabin plus Cyclophosphamid, Bendamustin oder Chlorambucil und
n) Fludarabin plus Cyclophosphamid, Bendamustin oder Chlorambucil, kombiniert mit einem Anti-CD20-Antikörper,
einen Rückfall erleidet.

9. Kit zur Verwendung gemäß Anspruch 8, wobei der PD-1-Inhibitor ein aus der Gruppe Nivolumab und Pembrolizumab ausgewählter Anti-PD-1-Antikörper ist.

10. Kombination zur Verwendung nach Anspruch 1, 6 oder 8, wobei der Mensch (i) refraktär gegenüber der mindestens einen Antikrebstherapie ist und (ii) nach der Behandlung mit der mindestens einen Antikrebstherapie einen Rückfall erleidet.

11. Kombination aus einem Btk-Inhibitor und einem Checkpoint-Inhibitor zur Verwendung bei der Sensibilisierung eines Menschen, der (i) refraktär gegenüber mindestens einer Chemotherapiebehandlung ist oder (ii) nach einer Behandlung mit Chemotherapie einen Rückfall erleidet, oder sowohl (i) als auch (ii), wobei der Btk-Inhibitor die chemische Struktur 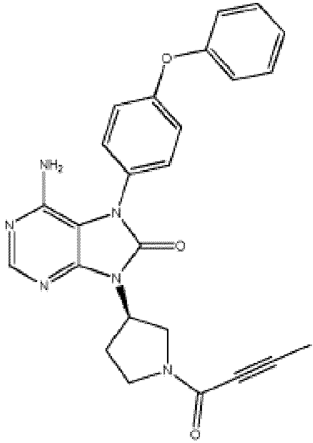 hat, wobei der Checkpoint-Inhibitor ein Inhibitor von PD-1 ist und wobei der Mensch gegenüber mindestens einer der Krebstherapien refraktär ist oder nach einer Behandlung mit mindestens einer Anti-Krebstherapie, ausgewählt aus der Gruppe von:
a) Fludarabin,
b) Rituximab,
c) Rituximab kombiniertmit Fludarabin,
d) Cyclophosphamid kombiniertmit Fludarabin,
e) Cyclophosphamid kombiniertmit Rituximab und Fludarabin,
f) Cyclophosphamid kombiniertmit Vincristin und Prednison,
g) Cyclophosphamid kombiniertmit Vincristin, Prednison und Rituximab,
h) eine Kombination aus Cyclophosphamid, Doxorubicin, Vincristin und Prednison,
i) Chlorambucil kombiniertmit Prednison, Rituximab, Obinutuzumab oder Ofatumumab
j) Pentostatin kombiniertmit Cyclophosphamid und Rituximab,
k) Bendamustin kombiniertmit Rituximab,
l) Alemtuzumab,
m) Fludarabin plus Cyclophosphamid, Bendamustin oder Chlorambucil und
n) Fludarabin plus Cyclophosphamid, Bendamustin oder Chlorambucil, kombiniert mit einem Anti-CD20-Antikörper,
einen Rückfall erleidet.

12. Kombination zur Verwendung nach Anspruch 1, 6, 8 oder 11, wobei der Anti-CD20-Antikörper ausgewählt ist aus der Gruppe Rituximab, Ofatumumab und Obinutuzumab.

## Revendications

1. Combinaison d'une quantité thérapeutiquement efficace d'un inhibiteur BTK et d'une quantité thérapeutiquement efficace d'un inhibiteur de point de contrôle pour une utilisation dans le traitement du cancer chez un humain, dans laquelle
- l'inhibiteur BTK a la structure chimique : 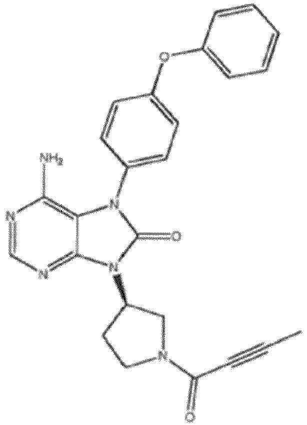
- l'inhibiteur de point de contrôle est un inhibiteur PD-1, et
- l'individu est réfractaire à au moins une des thérapies anticancéreuses, ou est en rechute après un traitement avec au moins une thérapie anticancéreuse du groupe comprenant :
a) fludarabine,
b) rituximab,
c) rituximab associé à la fludarabine,
d) cyclophosphamide associé à la fludarabine,
e) cyclophosphamide associé au rituximab et à la fludarabine,
f) cyclophosphamide associé à la vincristine et à la prednisone,
g) cyclophosphamide combiné à la vincristine, à la prednisone et au rituximab,
h) combinaison de cyclophosphamide, doxorubicine, vincristine et prednisone,
i) chlorambucil associé à la prednisone, au rituximab, à l'obinutuzumab ou à l'ofatumumab,
j) pentostatine associée au cyclophosphamide et au rituximab,
k) bendamustme associé au rituximab,
l) alemtuzumab,
m) fludarabine plus cyclophosphamide, bendamustine ou chlorambucil, et
n) fludarabine plus cyclophosphamide, bendamustine ou chlorambucil, en association avec un anticorps anti-CD20.

2. Combinaison à utiliser selon la revendication 1,
dans laquelle
l'inhibiteur BTK et/ou l'inhibiteur de point de contrôle doit être administré par voie intraveineuse, intramusculaire, parentérale, nasale ou orale.

3. Combinaison à utiliser selon la revendication 1,
dans laquelle
l'inhibiteur BTK doit être administré avant, après ou en même temps que l'inhibiteur de point de contrôle.

4. Combinaison à utiliser selon la revendication 1,
dans laquelle
le cancer est dans le groupe constitué par : malignité hématologique, leucémie, lymphome et myélome multiple.

5. Combinaison à utiliser selon la revendication 1,
dans laquelle
l'inhibiteur PD-1 est un anticorps anti-PD-1 du groupe comprenant : le nivolumab et le pembrolizumab.

6. Produit de fabrication destiné à être utilisé dans le traitement du cancer chez un humain comprenant :
(i) une forme d'unité posologique d'un inhibiteur BTK, dans laquelle l'inhibiteur BTK est la 6-amino-9-[(3R)-1-(2butynoyl)-3-pyrrolidinyl]-7-(4-phénoxyphényl)-7,9-dihydro-8H-purin-8-one, et
(ii) une forme d'unité posologique d'un inhibiteur de point de contrôle, dans laquelle l'inhibiteur de point de contrôle est un inhibiteur PD-1, et
(iii) une étiquette contenant des instructions pour l'utilisation de l'inhibiteur BTK et de l'inhibiteur de point de contrôle dans le traitement du cancer,
- l'humain étant réfractaire à au moins une des thérapies anticancéreuses, ou est en rechute après un traitement avec au moins une thérapie anticancéreuse dans le groupe comprenant :
a) fludarabine,
b) rituximab,
c) rituximab associé à la fludarabine,
d) cyclophosphamide associé à la fludarabine,
e) cyclophosphamide associé au rituximab et à la fludarabine,
f) cyclophosphamide associé à la vincristine et à la prednisone,
g) cyclophosphamide associé à la vincristine, à la prednisone et au rituximab,
h) association de cyclophosphamide, doxorubicine, vincristine et prednisone,
i) chlorambucil associé à la prednisone, au rituximab, à l'obinutuzumab ou à l'ofatumumab,
j) pentostatine associée au cyclophosphamide et au rituximab,
k) bendamustine associée au rituximab,
l) alemtuzumab,
m) fludarabine plus cyclophosphamide, bendamustine ou chlorambucil, et
n) fludarabine plus cyclophosphamide, bendamustine ou chlorambucil, en association avec un anticorps anti-CD20.

7. Produit de fabrication destiné à être utilisé selon la revendication 6, dans lequel l'inhibiteur PD-1 est un anticorps anti-PD-1 choisi dans le groupe du nivolumab et du pembrolizumab.

8. Kit destiné à être utilisé dans le traitement du cancer chez un humain, le kit comprenant :
(i) une composition pharmaceutique comprenant un inhibiteur BTK ayant la structure suivante : 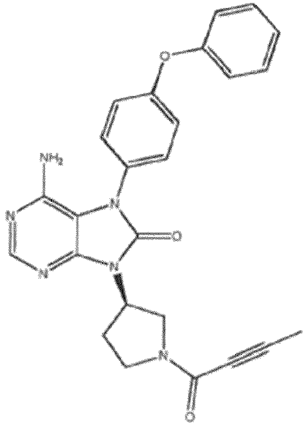
(ii) une composition pharmaceutique comprenant un inhibiteur de point de contrôle, cet inhibiteur de point de contrôle étant un inhibiteur de PD-1,
l'humain étant réfractaire à au moins une des thérapies anticancéreuses, ou en rechute après un traitement avec au moins une thérapie anticancéreuse dans le groupe comprenant :
a) fludarabine,
b) rituximab,
c) rituximab associé à la fludarabine,
d) cyclophosphamide associé à la fludarabine,
e) cyclophosphamide associé à du rituximab et de la fludarabine,
f) cyclophosphamide associé à la vincristine et à la prednisone,
g) cyclophosphamide combiné à la vincristine, à la prednisone et au rituximab,
h) combinaison de cyclophosphamide, doxorubicine, vincristine et prednisone,
i) chlorambucil associé à la prednisone, au rituximab, à l'obinutuzumab ou à l'ofatumumab,
j) pentostatine associée au cyclophosphamide et au rituximab,
k) bendamustine associée au rituximab,
l) alemtuzumab,
m) fludarabine plus cyclophosphamide, bendamustine ou chlorambucil, et
n) fludarabine plus cyclophosphamide, bendamustine ou chlorambucil, associés à un anticorps anti-CD20.

9. Kit à utiliser selon la revendication 8,
dans lequel
l'inhibiteur PD-1 est un anticorps anti-PD-1 choisi dans le groupe du nivolumab et du pembrolizumab.

10. Combinaison à utiliser selon la revendication 1, 6 ou 8,
dans laquelle
l'humain est :
(i) réfractaire à au moins une thérapie anticancéreuse, et
(ii) en rechute après le traitement avec au moins une thérapie anticancéreuse.

11. Combinaison d'un inhibiteur BTK et d'un inhibiteur de point de contrôle pour une utilisation dans la sensibilisation d'un humain qui est :
(i) réfractaire à au moins un traitement de chimiothérapie, ou
(ii) en rechute après un traitement par chimiothérapie, ou à la fois (i) et (ii),
l'inhibiteur BTK ayant la structure chimique suivante : 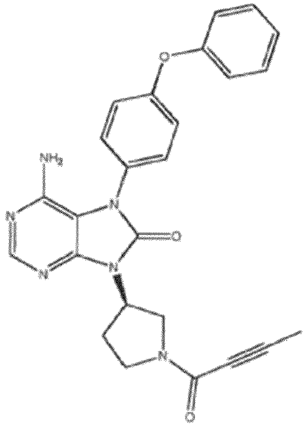
- l'inhibiteur de point de contrôle étant un inhibiteur de PD-1,
et l'humain étant réfractaire à au moins une des thérapies anticancéreuses, ou en rechute après un traitement avec au moins une thérapie anticancéreuse dans le groupe comprenant :
a) fludarabine,
b) rituximab,
c) rituximab associé à la fludarabine,
d) cyclophosphamide associé à la fludarabine,
e) cyclophosphamide associé au rituximab et à la fludarabine,
f) cyclophosphamide associé à la vincristine et à la prednisone,
g) cyclophosphamide associé à la vincristine, à la prednisone et au rituximab,
h) combinaison de cyclophosphamide, doxorubicine, vincristine et prednisone,
i) chlorambucil associé à la prednisone, au rituximab, à l'obinutuzumab ou à l'ofatumumab,
j) pentostatine associée au cyclophosphamide et au rituximab,
k) bendamustine associée au rituximab,
l) alemtuzumab,
m) fludarabine plus cyclophosphamide, bendamustine ou chlorambucil, et
n) fludarabine plus cyclophosphamide, bendamustine ou chlorambucil, en association avec un anticorps anti-CD20.

12. Combinaison à utiliser selon la revendication 1, 6, 8 ou 11,
dans laquelle
l'anticorps anti-CD20 est choisi dans le groupe comprenant : rituximab, ofatumumab et obinutuzumab.
